(19) Europäisches Patentamt
European Patent Office
Office européen des brevets

(11) **EP 3 954 290 A1**

(12) **EUROPEAN PATENT APPLICATION**

(43) Date of publication:
**16.02.2022  Bulletin 2022/07**

(21) Application number: **20190368.9**

(22) Date of filing: **10.08.2020**

(51) International Patent Classification (IPC):
*A61B 5/16* *(2006.01)*     *A61B 5/374* *(2021.01)*
*G16H 20/10* *(2018.01)*    *G16H 50/20* *(2018.01)*
*A61M 21/00* *(2006.01)*    *A61B 5/01* *(2006.01)*
*A61B 5/024* *(2006.01)*    *A61B 5/0245* *(2006.01)*
*A61B 5/08* *(2006.01)*     *A61B 5/145* *(2006.01)*

(52) Cooperative Patent Classification (CPC):
(C-Sets available)
**A61B 5/165; A61B 5/374; A61B 5/6803;**
**A61B 5/7435; A61M 21/00; G16H 20/10;**
**G16H 50/20; G16H 50/30;** A61B 5/01;
A61B 5/02405; A61B 5/02416; A61B 5/0245;
A61B 5/0816; A61B 5/14542; A61B 5/163;    (Cont.)

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(71) Applicant: **Oncomfort SA**
**1300 Wavre (BE)**

(72) Inventors:
• **Jooris, Diane**
  **3080 Tervuren (BE)**
• **Huyghe, Mario**
  **8792 Desselgem (BE)**
• **Toussaint, Clémence**
  **4210 Burdinne (BE)**

(74) Representative: **De Clercq & Partners**
**Edgard Gevaertdreef 10a**
**9830 Sint-Martens-Latem (BE)**

(54)    **A METHOD AND SYSTEM FOR MEASURING A LEVEL OF ANXIETY**

(57)    There is described a method and system for measuring a level of anxiety. Measured data comprising EEG data collected from a parietal (P) EEG electrode is received. A group 8 indicator based on a power, P-power (dt), associated with a delta-theta frequency band, dt, within a delta-theta frequency range is extracted. Based on said group 8 indicator, a level of anxiety, LoA, is determined which is a value indicative for the level of anxiety in the subject.

FIG. 3

**(Cont. next page)**

(52) Cooperative Patent Classification (CPC): (Cont.)
A61B 5/352; A61B 5/389; A61B 5/398;
A61B 5/4839; A61B 5/4848; A61B 5/7246;
A61B 2560/0223; A61M 2021/0016;
A61M 2021/0022; A61M 2021/0027;
A61M 2021/005; A61M 2205/332;
A61M 2205/3375; A61M 2205/502;
A61M 2205/507; A61M 2205/70; A61M 2230/04;
A61M 2230/06; A61M 2230/10; A61M 2230/14;
A61M 2230/205; A61M 2230/30; A61M 2230/40;
A61M 2230/42; A61M 2230/50; A61M 2230/60

C-Sets
A61M 2230/04, A61M 2230/005;
A61M 2230/06, A61M 2230/005;
A61M 2230/10, A61M 2230/005;
A61M 2230/14, A61M 2230/005;
A61M 2230/205, A61M 2230/005;
A61M 2230/30, A61M 2230/005;
A61M 2230/40, A61M 2230/005;
A61M 2230/42, A61M 2230/005;
A61M 2230/50, A61M 2230/005;
A61M 2230/60, A61M 2230/005

## Description

### Field of the invention

The present invention is in a field of a computer-implemented method for automatically monitoring a level of anxiety as experienced by a subject.

### Background to the invention

Anxiety and, for example, the impact of anxiety on patient comfort and the effectiveness of medical treatments is a well-known and universal problem. Anxiety management is thus an important branch of medicine. However prior art methods for assessment of the level of anxiety a subject is experiencing have important drawbacks and are not able to provide for an efficient, reliable, robust, objective and reproducible indication of the level of anxiety as experienced by a subject. This problem is immediately apparent when the subject is not capable of providing for a reliable or clear description of the level of anxiety experienced, such as for example in the case of non-verbal children, persons with a disability, ... .

A patient's self-reported anxiety is currently a critical tool in measurement of a level of anxiety. Such self-reported anxiety tools provide for a measure of the level of anxiety as experienced by the subject. Such self-reported anxiety tools are known, such as for example the State-Trait Anxiety Inventory or STAI, BECK anxiety inventory, .... Such a level of anxiety as experienced by the subject, is defined as a psychophysiological state that reflects anxious reactions, which could for example be triggered by an anxiety inducing event or situation. It is known, for example from Leal et al., 2017, that anxiety is a multi-dimensional concept characterized by for example a cognitive-worry dimension as well as an autonomic-emotional dimension. It is this clear that the level of anxiety experienced by a subject refers to the strength of the psychophysiological reaction, for example to an anxiety inducing event or situation. Further, it is known that there are individual differences with respect to a level of anxiety as experienced by a subject when subjected to a particular anxiety inducing event. Such individual differences are for example an important characteristic of patients with anxiety disorders. There is thus a need to determining the level of anxiety experienced by a subject and/or individual differences of the level of anxiety experienced by subjects in a reliable and efficient way.

Currently, in order to assess the level of anxiety as experienced by the patient, use is made of self-reporting tools, such as for example the Spielberger State-Trait Anxiety Inventory or STAI. However, it is clear that such manual tools that rely on self-reported assessment of the patient are prone to undesired subjective bias and lack efficiency, reliability, precision and reproducibility. Further such self-reporting tools lack the ability to take into account the multi-dimensional aspect of anxiety. Typically, such self-reporting tools are extensive and burdensome to administer and complex and intensive to process because of a large number of questions.

Accurately and reliably measuring anxiety is important in a medical setting as it for example helps to determine an accurate diagnosis, a treatment plan, and evaluate the effectiveness of treatment. Additionally, there is a growing problem of anxiety treatment overdosing, such as for example anxiolytic overdosing, and care workers are in need of more reliable tools in order to provide for a safe and effective anxiety treatment. A robust and reliable measurement of the level of anxiety of a subject could thus provide for a safer and more effective anxiety treatment, thereby reducing the risk for overdosing and/or underdosing, and thus increasing the quality of life of the subject in a safe way. It is further clear that anxiety and the treatment thereof has an impact on medical outcomes and patient recovery, and anxiety has an impact health-related effects such as for example fatigue, nausea, insomnia, pain, for example as experienced by a subject before, during and/or after a treatment, such as for example a treatment including surgery and/or any other suitable medical and/or non-medical treatment.

In general, there is a need to more objectively measure, monitor, trend and predict the level of anxiety of a subject, both in the context of a treatment or outside the context of a treatment.

### Summary

According to a first aspect, there is provided a computer-implemented method for measurement of a level of anxiety, the method comprising the steps of:

-   receiving measured data comprising EEG data collected from a parietal (P) EEG electrode configured for collection of P-EEG electrode data from the scalp anatomical region corresponding to a parietal lobe of a subject;
-   extracting from the EEG data collected from the P-EEG electrode:

    -   a group 8 indicator based on a power, P-power (dt), associated with a delta-theta frequency band, dt, within a delta-theta frequency range;

- determining, based on said group 8 indicator, a level of anxiety, LoA, which is a value indicative for the level of anxiety in the subject.

This allows for an automated, efficient, accurate, reproducible, and objectivized determination and/or monitoring of the level of anxiety as experienced by the subject. In this way a simple but effective measurement, which takes into account the cognitive dimension of anxiety, has been found. This improved anxiety measurement then helps in determining a more accurate diagnosis, an improved treatment plan, and an improved evaluation the effectiveness of a treatment. This also reduces the risk for overdosing and/or underdosing during treatment of anxiety, and thus increases the quality of life of a subject in a safe way. In this way the use of pharmacological anxiolytics and/or non-pharmacological anxiety treatments can be optimized.

It should be clear that according to some embodiments, this method is limited to merely obtaining information, such as data and physical quantities from the living human body and is not directed to finding any significant deviation, i.e. a symptom during a comparison of the collected data with standard values and is not directed to the attribution of such a deviation to a particular clinical picture, i.e. the deductive medical decision phase. Preferably, according to some embodiments, the method is performed on measured data, received, after it has been collected, or in other words, the method excludes a step of collection of data practised on the human body. According to some embodiments the computer-implemented method only concerns obtaining information, for measurement of the level of anxiety as experienced by a subject, which is a psychophysiological quality of the subject. According to some embodiments, the computer-implemented method excludes any steps for curing of a disease or malfunction of the body, nor any step relating to a therapeutic effect on the body of the subject, nor any diagnostic step performed on the body of the subject.

It is clear that the level of anxiety as measured comprises at least a level of anxiety experienced by a subject as measured at the level of the cortex of the brain of the subject or in other words comprising a cognitive dimension. Anxiety, being a response involving a psychophysiological and emotional aspect, clearly involves such a cognitive dimension. It has been shown based on the experiments above that anxiety as measured above is able to capture this cognitive dimension and is not limited to measurement of only non-cognitive parameters. Anxiety is for example associated with increased activity in the amygdala, and the activity of the amygdala can be influenced by activity in the higher brain structure, for example including the anterior cingulate cortex and the pre-frontal cortex responsible for focused attention. It is thus clear that anxiety as experienced by a subject comprises a cognitive dimension, optionally in addition to any other suitable dimensions.

As will be explained in further detail below, there was only found a specific correlation for this specific combination of brain wave frequencies of data that was collected at this specific location with an EEG electrode. Further, calculating the power from this data, which can be collected from a single EEG electrode, can be accomplished in a simple and efficient manner.

According to an embodiment there is provided a method, wherein the delta-theta frequency band, dt, comprises one or more of the following:

- a frequency band encompassing both delta and theta brain waves;
- a frequency band extending from 0 Hz up to and including 8 Hz;
- a bandwidth of at least 2 Hz and a frequency band comprising at least slow theta brain wave extending from 3 Hz up to and including 6 Hz; and/or

wherein the delta-theta frequency range:

- comprises a frequency range encompassing both delta and theta brain waves; and/or
- extends from 1 Hz up to and including 6 Hz.

It is clear, that according to advantageous embodiments, the delta-theta frequency band for example comprise a combination of at least two, or preferably exactly two types of brainwaves.

According to an advantageous embodiment the delta-theta frequency band consists of a combination of:

- at least part of a delta frequency band; and
- at least part of a theta frequency band.

According to an advantageous embodiment the delta-theta frequency band consists of a combination of at least part of a delta frequency band and at least part of a theta frequency band.

In other words, the delta-theta (dt) frequency band comprises, and preferably consists of a combination of at least part of the delta frequency band and at least part of the theta frequency band.

It is clear that such a delta-theta frequency band comprising and/or consisting of a combination of two brain wave frequencies, or at least a part thereof, is different from a frequency band only comprising a single brain wave frequency band and different from a frequency band comprising more than two brain wave frequency bands.

It has been found that such a combined frequency band, consisting of two specific brain frequency bands, surprisingly provides for a better correlation with the level of anxiety, than each of the individual brain frequency bands, for data collected from the specific location of a P-EEG electrode.

According to an embodiment the method comprises the steps of:

- receiving measured data further comprising cardiovascular data
- extracting from the cardiovascular data:

  - a group 7 indicator based on a heart rate and/or heart rate variability, preferably a combination of heart rate and heart rate variability, from the cardiovascular data;

- determining the LoA based on said group 8 indicator and at least the group 7 indicator.

According to such embodiments in which the level of anxiety is measured by means of a combination of a specific signal extracted from EEG data, which is more directly linked to the cognitive dimension of anxiety, and one or more specific signals like heart rate, heart rate variability, breathing frequency, breathing variability, oxygen saturation, ... and/or other suitable specific embodiments of physiological parameters as mentioned below, which are more directly linked to the autonomic-emotional dimension provides for a specifically advantageous multidimensional measurement of the level of anxiety as experienced by a subject. Based on the specific signals extracted from both specific EEG data and at least one specific other signal from a specific physiological parameter thus allows for a measurement that takes into account the multi-dimensionality of anxiety, in which however the cognitive dimension as linked to the specific EEG data is essential in order to provide for a measurement that relates to the level of anxiety as experienced by the subject.

As will be explained in further detail below, a specific combination of heart rate and heart rate variability data, provides for a particularly advantageous and simple measurement with an improved correlation with the level of anxiety as experienced by a subject. However, it is clear that alternative embodiments are possible in which any other suitable cardiovascular data and/or combination thereof is used, such as for example cardiac activity data, hemodynamic activity data, ....

According to an embodiment the method comprises the steps of:

- receiving measured data further comprising respiratory data;
- extracting from the respiratory data:

  - a group 6 indicator based on respiration rate and/or a respiration variability and/or predetermined respiration patterns and/or oxygen saturation from the respiratory data;

- determining the LoA based on said group 8 indicator and at least the group 6 indicator.

According to an embodiment the method comprises the steps of:

- receiving measured data further comprising ocular movement data;
- extracting from the ocular movement data:

  - a group 9 indicator based on ocular movement rate and/or ocular movement amplitude and/or predetermined ocular movement patterns from the ocular movement data;

- determining the LoA based on said group 8 indicator and at least the group 9 indicator.

According to an embodiment:

- the ocular movement data comprises electrooculographic or EOG data; and/or
- the group 9 indicator is based on the detection of saccadic eye movements from the ocular movement data, and optionally one or more characteristics of the saccadic eye movements, such as frequency, amplitude, direction and/or predetermined saccadic eye movement patterns.

According to an embodiment the method comprises the steps of:

- receiving measured data further comprising
- muscular activity data;
- extracting from the muscular activity data:

  - a group 10 indicator based on muscular activity from the muscular activity data;

- determining the LoA based on said group 8 indicator and at least the group 10 indicator.

According to a preferred embodiment the muscular activity data comprises electromyography data or EMG data, and/or the group 10 indicator is based on electromyographic activity or EMG activity from the EMG data.
According to a preferred embodiment the EMG data is captured at two or more electrode locations such that global muscle tension can be determined and locally isolated artefacts can be detected and removed.
According to an embodiment the method comprises the steps of:

- receiving measured data further comprising
- body temperature data;
- extracting from the body temperature data:

  - a group 11 indicator based on a body temperature from the body temperature data;

- determining the LoA based on said group 8 indicator and at least the group 11 indicator.

According to an embodiment the method comprises the steps of:

- receiving reference data comprising measured data of a subject and/or a population correlated to at least one predetermined reference LoA;
- determining at least one correlation of at least one predetermined reference LoA and at least one group indicator extracted from the reference data; and
- scaling and/or indexing a subsequently measured LoA with respect to the at least one predetermined reference LoA by means of said at least one correlation, and optionally determining a level of anxiety index or LoAI therefrom.

According to a second aspect, there is provided a computer-implemented method for measuring and/or monitoring a level of anxiety of a subject, comprising the method for measurement of a level of anxiety according to the first aspect applied on a subject by receiving the measured data of a subject.
According to a third aspect, there is provided a computer-implemented method for measuring and/or monitoring and/or predicting and/or aggregating a level of anxiety in a subject before, during and/or after a treatment, wherein the method comprises the steps of:

- measurement of the LoA according to the first aspect, at at least two different points in time, preferably before, during and/or after the treatment;
- determining and/or monitoring and/or predicting and/or aggregating an evolution, preferably a treatment-induced evolution of the LoA, based on a comparison of at least two LoA measured at at least two different points in time; and/or
- aggregating a level of anxiety score or LoAS based on an aggregation of at least two LoA measured at at least two different points in time.

According to an embodiment, the method comprises the step of:

- determining the treatment-induced evolution of the LoA, based on a comparison of the LoA measured during and/or after the treatment with the LoA measured before the treatment.

According to an embodiment the treatment is an anxiety reducing treatment and the method comprises the step of:

- determining and/or monitoring the efficacy of the anxiety reducing treatment based on the treatment-induced evolution of the LoA; and/or
- optimizing and/or adapting an anxiety reducing treatment based on the treatment-induced evolution of the LoA.

According to a fourth aspect, there is provided a computer-implemented method for determining a treatment and/or determining an adjustment to a treatment and/or determining an intensity of a treatment, wherein the method comprises the steps of:

- measuring a LoA according the first aspect;
- determining a treatment and/or determining an adjustment to a treatment and/or determining an intensity of a treatment based on the measured LoA.

According to a fifth aspect there is provided a computer program product and/or a computer-readable storage medium and/or a computer-readable data carrier and/or a data carrier signal comprising instructions which, when executed by a computer, cause the computer to carry out the steps of the method according to any of the preceding aspects and their embodiments.

According to a sixth aspect, there is provided a system configured to measure a level of anxiety according to the any of the preceding aspects, the system comprising:

- a monitoring apparatus configured to obtain measured data comprising electroencephalogram, EEG, data comprising P-EEG data collected from a P-EEG electrode;
- a controller module configured for:

  - receiving the measured data from the monitoring apparatus;
  - extracting from the EEG data:

    - a group 8 indicator based on a power, P-power (dt), associated with a delta-theta frequency band, dt, within a delta-theta frequency range;

  - determining, based on said group 8 indicator, the level of anxiety, LoA, which is a value indicative for the level of anxiety in the subject.

According to an embodiment there is provided a system, wherein the monitoring apparatus comprises, for obtaining measured data, one or more of:

- one or more parietal (P) EEG electrodes configured for collection of P-EEG electrode data from the scalp anatomical region corresponding to a parietal lobe of the subject,
- optionally, one or more further EEG electrodes configured for collection of electroencephalogram, ECG, data;
- optionally, one or more frontal (F) EEG electrodes configured for collection of F-EEG electrode data from the scalp anatomical region corresponding to a frontal lobe of the subject,
- optionally, one or more central (C) EEG electrodes configured for collection of C-EEG electrode data from the scalp anatomical region corresponding to a precentral and postcentral gyrus of the subject;
- optionally a cardiovascular data capturing unit;
- optionally a respiratory data capturing unit;
- optionally a muscular activity data capturing unit;
- optionally an ocular movement data capturing unit;
- optionally a body temperature data capturing unit; and/or

wherein the system further comprises a graphical user interface, GUI, configured to indicate numerically and/or graphically one or more of:

- at least two LoA and/or LoAI respectively measured at different points in time, preferably a historic, current and/or expected LoA and/or LoAI.
- the evolution of and/or ratio between and/or aggregation of at least two LoA and/or LOAI measured at different points in time;
- optionally an aggregated level of anxiety score or LoAS or LoAIS based on an aggregation of at least two LoA or LoAI measured at at least two different points in time;
- optionally, one or more components of the measured data, preferably one or more P-EEG electrode data.

It is clear that still further alternative embodiments are possible comprising for example one or more sensors and/or capturing units for one or more other electrophysiological and/or physiological measurements.

It is clear that alternative embodiments of the GUI are possible in which further data is displayed, such as for example ECG data, cardiovascular data, respiratory data, ocular movement data, EOG data, muscular activity data, EMG data, body temperature data, ....

It is clear that in the abovementioned embodiments of the method and/or systems any embodiments defined with reference to LoA, also refer to similar embodiments based on parameters derived from LoA, such as for example LoAl and/or LoAS.

**Figure Legends**

FIG. 1 shows an embodiment of an experiment for measuring the level of anxiety as experienced by the subject.
FIG. 2 is a graph indicating a correlation between self-reported anxiety and computed delta-theta power at P-EEG electrode.
FIG. 3 shows an embodiment of a computer-implemented method for measuring the level of anxiety.
FIG. 4 shows an embodiment of a computer-implemented method for measuring and/or monitoring a level of anxiety in a subject before, during and/or after a treatment.
FIG. 5 is a graph indicating the correlation between a level of anxiety and a combined heart rate.
FIG.. 6 to FIG. 13 illustrate examples of outputs to a graphical user interface.
FIG. 14 illustrates a wearable device incorporating an eye-mask incorporating a media renderer, electrodes and sensors.
FIG. 15 illustrates a rim of the eye-mask of the wearable device disposed with electrodes.
FIG. 16 illustrates rim of the eye-mask of the wearable device disposed with another configuration of electrodes and sensors.
FIG. 17 is similar to FIG. 14 devoid of a virtual reality viewer, headphones are present.
FIG. 18 is similar to FIG. 14 devoid of a virtual reality viewer and headphones.
FIG. 19 shows an exemplary visualization of an exemplary treatment session comprising four phases, as measured using LoAI.
FIG. 20 shows an exemplary visualization of a time domain response ECG signal and a heart rate and heart rate variability extracted therefrom.
FIG. 21 shows a heart rate variability for a subject with a higher level of anxiety than in FIG. 23.
FIG. 22 shows a heart rate variability for a subject with a lower level of anxiety than in FIG. 22.
FIG. 23 shows a box plot comparison of two embodiments of a group 7 indicator.
FIG. 24 shows correlation between self-reported anxiety and respiration rate.
FIG. 25 shows such correlation between the self-reported level of anxiety and respiration rate variability.
FIG. 26 shows an exemplary embodiment of time domain respiratory data, and Fig. 27 and FIG. 28 show such respiratory data for a subject at different levels of anxiety.
FIG. 29 and 30 show an exemplary embodiment of time domain ocular movement data for a subject at different levels of anxiety.
FIG. 31 shows an exemplary embodiment of time domain ocular movement data averaged for a plurality of subjects which is time-locked to a painful stimulus, at two different levels of anxiety.
FIG. 32 shows an exemplary embodiment of time domain muscular activity data of a subject at two different levels of anxiety.

**Detailed description of exemplary embodiments**

In order to generate experimental data, according to this particular embodiment, an experiment including a hypnotic treatment session was set up. However, it is clear that alternative embodiments are possible in which any other suitable treatment session is used and/or in which no use is made of a treatment session. This experiment resulted in a study with 12 healthy subjects, of which 10 were included in the subsequent analysis. The experiment comprised measurement of a level of anxiety by self-reporting of the subject using a validated questionnaire.

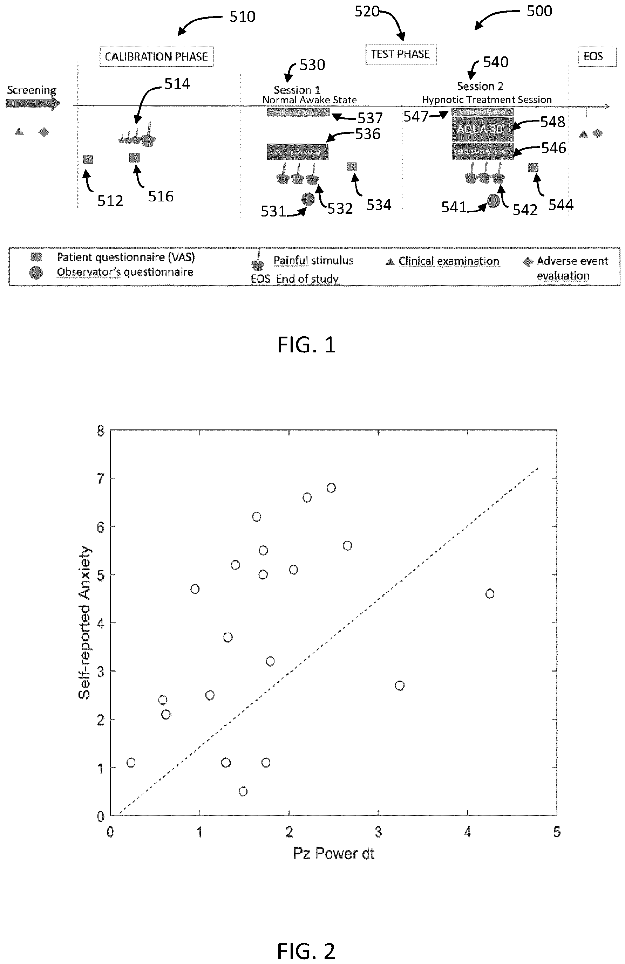

The setup of the embodiment of the experiment will be clarified by means of FIG. 1, which shows the different phases of the experiment. As shown, optionally, prior to and after the different phases of the experiment clinical examination and an adverse event evaluation was performed as part of a screening and/or the end of study, EOS, in order to assure the safety of the subjects undergoing the experiment and to assure a high level of quality with respect to the self-reported data.

During an initial calibration phase 510, at a first step 512 the subjects were asked to provide self-reported values for rating parameters as explained in further detail below. Subsequently at step 514, by using a stair-case method, the subjects received a sequence of transcutaneous electrical stimulus, or in other words a painful stimulus, of increasing intensity, so that in step 516 the subject could indicate when the experienced level of pain corresponded to a level of pain with a value of 8 on a Visual Analogue Scale or VAS pain scale. In other words, the intensity of the pain stimulus is calibrated for each subject individually using the stair-case method, by asking the subjects to indicate when they judged the experienced level of pain to corresponding to a value 8 on the pain scale, which corresponds to a level of pain qualified as "significantly painful and demanding some effort to tolerate". Such a pain scale for self-reporting of pain according to the embodiment of the experiment was determined by means of a Visual Analogue Scale or VAS, which is a measurement instrument that tries to measure a characteristic or attitude that is believed to range across a continuum of values and cannot easily be directly measured. According to this embodiment a VAS scale ranging from a value of 0 for "no pain" up to and including 10 for "severe or extreme amount of pain". Such instruments are often used in clinical research to measure the intensity of symptoms, such as for example, the level of pain as experienced by a patient. It is clear that alternative embodiments for self-reporting values are possible, such as for example the Numeric Rating Scale (NRS-11) is an 11-point scale for patient self-reporting of pain, which ranges from a value of 0 for "no pain" up to and including a value of 10 for "severe pain".

Similar as for the self-reported level of pain, at step 512, and 516 of the calibration phase 510, and as will be described in further detail below at further steps during the experiment, such as for example step 534 of the normal awake state 530 and step 544 of the hypnotic treatment session 540, patients were also asked to provide self-reported values for rating their experienced level of anxiety. According to this embodiment a VAS scale ranging from a value of 0 for "no anxiety" up to and including 10 for "severe or extreme amount of anxiety". It is clear that alternative embodiments for self-reporting values are possible.

As further schematically shown in FIG. 1, the calibration phase 510 was followed by a first phase 520, which was labelled as test phase 520 in Fig. 1, which comprises a first session 530 in a normal awake state of the subject. As schematically shown, during this first session 530, there were generated one or more pain stimuli, as calibrated during the previous calibration phase. As shown, according to this embodiment, there was for example generated a plurality, for example as shown in step 532 three pain stimuli that were qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. However, it is clear, that alternative embodiments of suitable time periods, during which the one or more pain stimuli 532 or any other suitable types of anxiety inducing stimuli are applied, are possible. During this time period, during which the pain stimuli 532 are applied, as shown at step 534, suitable sensors are configured to monitor and/or register physiological signals generated by the subject for provision to a suitable processor to generate measurement data corresponding to the state and/or activity of the body or bodily functions of the subject. Such physiological sensors provide an objective measure of physiological signals. Such physiological sensors provide for signals, that, when suitably processed, provide for measurement data based on recordings of electrical or other physiological signals produced by the brain, the heart, the muscles, the skin, ... of the subject. When the signals concern electrical signals, such as for example generated by the brain, heart, etc. then these signals can be referred to as electrophysiological signals. Embodiments of such electrophysiological sensors are for example sensors for measuring electrophysiological signals configured to measure electroencephalography or EEG, electrocardiography or ECG, electromyography or EMG, electrooculography or EOG, .... Other physiological signals for example comprise one or more of Breathing rate, Breathing rate variability, Blood Pressure or BP, Blood Volume Pressure or BVP, .... Electroencephalography or EEG is an electrophysiological monitoring method to record electrical activity of the brain, and can be referred to as a cognitive, electrophysiological signal, and is related to corticoidal signals. Other physiological and/or electrophysiological signals can be referred to as autonomic physiological signals such as for example EMG, which measures muscle activity; EDA or skin conductance which measures the hydration in the epidermis and/or dermis of the skin; EKG or ECG, which measures heart activity, such as for example heart rate, inter-beat-interval, heart rate variability, ... ; Electrooculogram, which measures eye movements, BVP, which measures blood pressure; Respiration sensors, which measure for example the rate of respiration, depth of breath, effort, temperature, air pressure during inhalation and/or exhalation, .... According to the embodiment shown, electroencephalogram (EEG) data, cardiovascular data (heart rate, heart rate variability, ...), electromyogram (EMG) data, respiratory data (respiration rate, respiration variability, predetermined respiration patterns, effort, temperature, and air pressure (inhalation/exhalation)), electrooculography or EOG and body temperature, were collected as measured data from each subject, however it is clear that alternative embodiments are possible comprising the monitoring of alternative cognitive electrophysiological and/or autonomic physiological signals as for example described in further detail below.

Subsequent to the time period 536 during which the measurements of the physiological signals were made, the subject was asked to provide self-reported values for the level of anxiety, etc. as experienced during the first session 530, for example by means a VAS patient questionnaire during step 534.

According to the embodiment of the experiment shown in FIG. 1, the test phase 520 subsequently comprises second session 540, which comprises an automated hypnotic treatment session 548 that was delivered using a system that comprises a virtual reality headset as will also be described in further detail below. However, it is clear that alternative embodiments are possible in which any other suitable treatment session is used and/or in which no use is made of a treatment session. The embodiment of the system, also referred to as a hypnotic package, is referred to as Sedakit, as manufactured by Oncomfort. The Sedakit was configured to the Aqua 30 minutes module embodiment of an automated hypnotic treatment session, which subjected the subject to an automatic hypnotic treatment session for a time period of 30 minutes at step 548. During this hypnotic treatment session 548, similar as described above, electroencephalogram (EEG), cardiovascular data, muscular activity data such as for example electromyogram or EMG data, respiratory data (respiration rate, respiration variability, predetermined respiration patterns, effort, temperature, and air pressure (inhalation/exhalation)), ocular movement data, such as for example electrooculography or EOG data and body temperature data, were collected as measured data from each subject, however it is clear that alternative embodiments are possible comprising the monitoring of alternative physiological signals. Also similar as described above, during the hypnotic treatment session 538, each subject was exposed to one or more transcutaneous painful stimuli, which also qualify as anxiety inducing events. For example, a sequence of a plurality of pain stimuli, such as for example shown as three painful stimuli 542. Similar as described above, according to this embodiment, the painful stimuli 542 that are applied during the hypnotic treatment session 538 were also qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities or any other suitable types of anxiety inducing stimuli are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. According to the embodiment of the experiment the external pain stimulus was provided in the form of an electric transcutaneous stimulus being provided at the fore-arm during the experiment, however it is clear that alternative embodiments of a suitable pain stimulus are possible. Similarly, as described above, at step 544, subsequently each subject was asked to rate anxiety as they experienced it during the hypnotic treatment session 548 by means of a patient questionnaire. Optionally, but preferably, during both session 1 530 in the normal awake state and session 2 540 with the hypnotic treatment session the subject is placed in a similar context. For example, in both sessions, preferably the subject is placed in a similar position, for example laying on chair. As further shown, for example the subject is experiencing similar sounds 537, 547, such as for example sounds of a hospital environment. Optionally, in addition to the patient questionnaire, there may be made use of an observer's questionnaire, for example assure the quality of the experiment and/or to provide for additional and/or alternative ratings for one or more of the self-reported ratings of anxiety.

During the experiment, for the EEG measurements, EEG electrodes were used that were placed so as to cover the scalp and so as to detect local voltage fluctuations resulting from localized regions of the brain. According to this embodiment, a high-density EEG was employed with 256 sensors or channels, however it is clear that alternative embodiments with a different number of sensors are possible. According to the embodiment of the experiment, electrodes were placed according to the predefined layout for a 256-channel Hydrocel Geodesic Sensor Network. It is further known to a person skilled in the art that EEG electrodes mainly measure neural activity that occurs at the upper layers of the brain, or in other words the neural activity of the cortex of the brain. During the experiment, for the EMG measurements sensor, a pair of surface EMG sensors were placed on a leg of each patient to as to detect the electric potential generated by muscle cells when then muscles become activated. However it is clear that alternative embodiments are possible in which EMG sensors are provided at any other suitable location, such as for example the face, arm, ... .It is however clear that according to alternative embodiments use can be made of other types of suitable EEG electrodes or sensors, EMG electrodes or sensors and/or any other suitable type of electrode or sensor for measuring EEG and/or EMG signals.

Below are the results of the above-mentioned experiment, according to the embodiment of FIG.1. As mentioned above the experiment 500 comprises a test phase 520 with a "normal awake state" 530 and a "Hypnotic treatment session" for which both self-reported data with respect to the level of anxiety were collected in steps 538, 548 for each subject that was subjected to one or more calibrated pain stimuli, which also qualify as calibrated anxiety inducing events. The results of the experiment of FIG.1 show measurable and statistically significant effect of the hypnotic treatment session 540 versus the normal awake state 530 regarding, the level of anxiety, more specifically a reduction of the level of anxiety as illustrated by means of Table 1 below.

Table 1 below, shows a Wilcoxon signed-rank test of the self-reported data for subjects during the experiment of FIG. 1 during the normal awake state 530 and during the hypnotic treatment session 540:

| Self-reported data 534, 544 | Normal awake state 530 Mean ±SD Median [IQR] | Hypnotic treatment session 540 Mean ±SD Median [IQR] | p-value | Adjusted p-value (Bonferroni correction) |
|---|---|---|---|---|
| Self-reported level of Anxiety | 4.92 (1.74) 5.55 [1.8] | 3.16 (1.77) 3.1 [2.95] | 0.0004 | 0.0024 |

It should first be noted that, in reaction to the application of the pain stimuli during the normal awake state 530, the self-reported level of anxiety has increased with respect to the level of anxiety as reported before the application of the pain stimuli. It is thus clear that the pain stimuli thus also function as an anxiety inducing stimulus or an anxiety inducing event. This is confirmed by the measurement of a self-reported level of anxiety before the normal awake state 530, which had as mean +/- SD: 2,65 (2.06) and as Median [IQR]: 2.15 [3.55], with a p-value = 0.002 and adjusted p-value =0.008. In between the Normal awake state 530 and the Hypnotic treatment session 540, there was a pause or a break, and before the Hypnotic treatment session 540, also there was performed a measurement of the self-reported level of anxiety, which had as mean +/- SD: 2,79 (2.21) and as Median [IQR]: 2.65 [1.25], with a p-value = 0.53 and adjusted p-value = 1.

It is thus clear that, according to the embodiment described above, in which during both the normal awake state 530 and the hypnotic treatment session 540, pain stimuli of an identical intensity were applied, or in other words, identical pain conditions were experienced by the subjects during application of the pain stimuli of identical intensity, or in other words an identical anxiety inducing events and/or stimuli, the self-reported level of Anxiety is significantly different in both sessions 530, 540. As for example shown, the mean of the value for self-reported anxiety in the normal awake state 530 is 4.92, while in the hypnotic treatment session 540 it was reported to be 3.16. In other words, a reduction of more than 30% with respect to the normal awake state. It is thus clear that the self-reported level of anxiety corresponds to a level of anxiety as experienced by the subject, as clearly, the self-reported values for the level of anxiety triggered by an identical external pain stimulus are modulated by the modified state of consciousness caused by the hypnotic treatment session. In other words, the self-reported data for the level of anxiety are an indicator for the level of anxiety as experienced by the subject, or in other words the level of experienced anxiety. As the self-reported data relating to anxiety are triggered by the painful stimuli, it is clear that these painful stimuli can be considered as an anxiety inducing event, or in other words an adverse situation at a specific moment in time, and thus the self-reported data relates to the level of anxiety of the subject as experienced with respect to the painful, anxiety inducing stimuli. Further it should be noted that the experiment was set up with a first phase in the normal awake state 530 with increased levels of anxiety as compared to prior to the experiment, and still during the subsequent second phase in during the hypnotic treatment session 540, again reduced levels of anxiety are reported. A predetermined rest time between the normal awake state 530 and the hypnotic treatment session 540 was included.

Additionally, as shown in further detail in table 2 below, there was found to be no statistically significant correlation between the self-reported level of anxiety and the stimulation intensity of the pain stimulus, or in other words the intensity of the applied transcutaneous electrical stimulus. As already mentioned above, the self-reported level of anxiety thus corresponds to the level of anxiety as experienced by the subject, and is thus not directly correlated to the intensity of the external pain stimulus, or in other words the intensity of the anxiety inducing event itself, but relates to the level of anxiety as cognitively experienced by the subject. In other words, the self-reported level of anxiety clearly relates to the experience of anxiety as it appears in the cognitive experience and optionally the autonomic physiological experience of the subject.

Table 2 below shows the Spearman correlation for both hypnotic treatment session 540 and normal awake state 530:

| | Self-reported level of Anxiety |
|---|---|
| Stimulation Intensity of the electrical pain stimulus | rs = -0.205 p = 0.334 |

In Table 2, the r is the general correlation coefficient. The rs is the spearman correlation coefficient. It can take a range of values from +1 to -1. A value of 0 indicates that there is no association between the two variables. A value greater than 0 indicates a positive association; that is, as the value of one variable increases, so does the value of the other variable. A value less than 0 indicates a negative association; that is, as the value of one variable increases, the value of the other variable decreases. The p is the p-value associated with the statistical test to evaluate whether the correlation is statistically significant and not just a coincidence. Generally, a p-value smaller than 0.05 is considered as statistically significant. When a p-value is closer to zero, this shows a higher statistical relevance, or in other words a better correlation.

Results from the analysis of the EEG measurement data generated during experiment 500 in combination with the self-reported data allowed associations to made between localized brain structures/networks and the cognitive experience of the level of anxiety. Identification of those localized structures/networks associated with the cognitive experience of anxiety allows for an objective monitoring of the level of anxiety as experienced by the subject. It is clear, as explained in further detail below, that by means of the above experiment 500, where the same calibrated external pain stimulus, which also qualifies as a calibrated anxiety inducing event or stimulus, was applied, during a normal awake state and during a hypnotic treatment session, that the EEG measurements, which differed during the normal awake state and during the hypnotic treatment session, and which were correlated to the self-reported level of anxiety of the subjects, thus provide for a measurement of the a level of anxiety as experienced by the subject, as the calibrated external pain stimulus remained constant, and thus only the cognitive experience of the level of anxiety was changed during the hypnotic treatment session as compared to the normal awake state. As already mentioned above, the self-reported level of anxiety thus corresponds to the level of anxiety as experienced by the subject, and is thus not directly correlated to the intensity of the external pain and/or anxiety inducing stimulus, or in other words the intensity of the anxiety inducing event itself, but relates to the level of anxiety as cognitively experienced and optionally physiologically experienced by the subject.

*Hypnotic treatment session results - measured data (EEG) - frequency domain*

In the brain, several types of brain activity occur at the same time, which is also referred to as parallel processing. Specific types of brain activity can be correlated to a specific frequency band in the measured EEG data during the experiment. In order to explore the different types of brain activity, use can be made of a suitable processing of the EEG measurement data generated during the experiment 500, by transforming the time domain signals into the frequency domain, for example by means of a fast Fourier transform or FFT operation. From such frequency domain signals, then for example the power of a specific signal frequency band can be determined, where power for example refers to a square of the amplitude of the frequency domain signals within a specified in a frequency range associated with one or more particular brain waves, such as described in further detail below. According to the embodiments described below, the frequency bands could for example comprise a combination of at least two, or exactly two types of brainwaves, such as for example a delta-theta frequency band. This means that such delta-theta frequency band consists of a combination of at least part of a delta frequency band and at least part of a theta frequency band. This means that such a delta-theta frequency band consists of a combination of at least part of a delta frequency band and at least part of a theta frequency band.

In the EEG data of the measured data during the experiment 500, there were found significant power differences in a specific delta-theta (dt) frequency band, comprising a combination of two different types of brain waves, when comparing the measured data of the subject during the hypnotic treatment session 540, which is an embodiment of a non-pharmacologically induced modified state of consciousness, as compared to a normal awake state 530, and which relates to two different self-reported levels of anxiety as experienced by the subject:

- A P-power(dt) which is decreased during the hypnosis treatment session 540 with respect to the normal awake state 530 in the 1-6 Hz frequency band, which is an embodiment of a delta-theta, dt, frequency band within the delta-theta frequency range, located around the Parietal lobe (P), which is also referred to as the early component. As will be explained in further detail below, such EEG measurement data can be used as part of a group 8 indicator for the level of anxiety of a subject, and more particularly the experienced level of anxiety of a subject. It is clear from the above-mentioned description that also self-reported level of anxiety is also significantly different during the hypnosis treatment session 540 with respect to the normal awake state 530.

The early component refers to the P-power(dt) decrease that was measured in a time period from 0.1 to 0.5 sec after the pain stimulus onset or the onset of any other suitable anxiety inducing event. According to this specific embodiment, in which there is an external pain stimulus, which serves as an anxiety inducing event, the power was calculated from -0.5s to 1.5s from with reference to the stimulus onset, or in other words the power was calculated in a time window of 2s including the stimulus onset. It is however clear that alternative embodiments are possible, in which for example a time window of at least 0.1s, for example a time window in the range of 0,5s up to and including 60s, or any other suitable time window is used, at any other suitable point in time with respect to the stimulus onset, as long as the time window includes at least a portion at a point in time after the stimulus onset. According to still further embodiments, such as for example during continuous power measurements, not related to a specific external stimulus, the power can be continuously calculated based on overlapping time windows of for example 2s, or of any other suitable time period, such as for example at least 0.1s, or in the range of 0.5s up to and including 60s, ....

The abovementioned results are the first identifying brain frequency band differences between an automated hypnosis treatment session and a normal awake state, which relates to two different self-reported levels of anxiety as experienced by the subject, especially for a brain frequency band comprising a combination of two brain waves such as for example delta and theta brainwaves. It is clear that these results are also the first at identifying this for any other suitable treatment sessions, for example pharmacological and/or non-pharmacological treatment sessions, and/or in which no use is made of a treatment session. It is clear that this is also the first identification of brain frequency band differences, especially a brain frequency band comprising a combination of two brain waves, with respect to different levels of anxiety as experienced by the subject. As already mentioned above, it is clearthat such a frequency band comprising and/or consisting of a combination of two brain wave frequencies, or at least a part thereof, is different from a frequency band only comprising a single brain wave frequency band and different from a frequency band comprising more than two brain wave frequency bands.

From the measured EEG data during the experiment 500, there was extracted power(dt) at EEG electrode P, and the following results were obtained:

- The self-reported level of anxiety was correlated (spearman) with computed group 8 indicator, or in other words P-power (dt) at EEG electrode P (rs = 0.51, p = 0.022). FIG. 2 shows the correlation between the self-reported level of anxiety and the computed P-power (dt) at EEG electrode P from the measured data during the experiment 500, for the subjects that took part in experiment 500 as described above. It is thus clear that the specific group 8 indicator, P-power (dt) is correlated to the self-reported value for the level of anxiety;
- The group 8 indicator, or in other words, the P-power(dt) was significantly lowered during the hypnosis treatment session 540 as compared to the normal awake state 530, as calculated from the EEG measurement data at the EEG electrode location P (0.84 mV during normal awake state 530 vs 0.44 mV during the hypnosis treatment session 540, p = 0.027). This is consistent with the above-mentioned correlation between the group 8 indicator, P-power (dt) and the self-reported value for the level of anxiety. An embodiment of this experimental data is for example shown in FIG. 2.

It should be clear that the P-power (dt) calculated from the combined brainwave frequency band delta-theta, surprisingly provides for a substantially higher correlation to the self-reported level of anxiety of the each of its composing individual brainwave frequency bands, i.e. delta and theta. The self-reported level of anxiety was correlated (spearman) with P-power (delta), i.e. the same power calculation for the individual delta brain wave frequency band, at the same EEG electrode P, with an rs = 0.09 and a p-value = 0.80. The self-reported level of anxiety was correlated (spearman) with P-power (theta), i.e. the same power calculation for the individual theta brain wave frequency band, at the same EEG electrode P, with an rs = 0.24 and a p-value = 0.4. It is thus clear that the P-power of the specific delta-theta combined frequency band provides for a surprising and unexpectedly high level of correlation, which could not have been foreseen based on either of the individual frequency band components delta and theta.

The above experimental data generated during the experiment 500, which comprises both measurement data, such as EEG data, as well as self-reported data, and which could also be referred to as response data, confirms the existence of a correlation between self-reported level of anxiety and a power of a specific combined brain wave frequency band at a specific location, P-power (dt), associated with a delta-theta frequency band, dt, which according to this embodiment extends from 1Hz up to and including 6Hz, also referred to as a group 8 indicator. However, as already mentioned above, it is clear that alternative embodiments are possible in which the frequency band comprises a combination of both delta brainwaves and theta brainwaves.

It was determined from the experimental data above that, surprisingly, only measured data received from the P EEG electrodes show a certain level of correlation of power (dt) with the self-reported level of anxiety. In the current experiment there was found no correlation at different electrode locations such as for example the C and F electrode location. The experimental data for the C-EEG electrode, provided a C-power(dt) with an rs=-0.09 with a p-value=0.69 for the correlation with the self-reported level of anxiety. Similarly, the experimental data for the F-EEG electrode, provided for a F-power(dt) with an rs=0.18 with a p-value=0.44 for the correlation with the self-reported level of anxiety. It is thus clear, that only the EEG measurement data resulting from electrodes at a parietal location provided for a sufficiently high level of correlation with the level of anxiety, while the EEG measurement data from electrodes at other locations, such as the central and frontal location, did not provide for such a sufficiently high level of correlation, or in other words, there was no or a level of correlation that was very low. According to the embodiment described here the absolute value of the rs value at the parietal location was greater than or equal to 0.45 and the absolute value of the rs value at other locations was lower than or equal to 0.20 .

It is clear that according to alternative embodiments, an alternative external pain and/or anxiety stimulus could be provided. It is clear that instead of and/or in addition to measurement of the level of anxiety in reaction to an external pain and/or anxiety stimulus, the experimental data can also be used for measurement of a level of anxiety caused by internal events or states of the subject, which for example could relate to anxiety experienced, which is not caused by an external event, such as an external pain and/or anxiety stimulus, being applied. It is clear that alternative embodiments of the experiment are possible, in which the P-power(dt) is determined for any other suitable frequency band comprising a combination of both delta brain waves and theta brainwaves, or at least part thereof, such as for example any suitable frequency band extending in the frequency range of 0Hz up to and including 8 Hz.

These findings have been advantageously applied in a computer-implemented method for measurement of a level of anxiety. Based on the insight that there exists a correlation between P-power(dt) and self-reported measurements of the level of anxiety, it now becomes possible to more objectively measure a level of anxiety, even in cases where self-reported anxiety measures are not possible or available, such as for example when the subjects are in a state in which they are unable or experience difficulties to communicate, when the subjects are young children, ... It is clear that the Level of Anxiety, or LoA is an indicator of the strength of the psychophysiological response to an anxiety inducing event or situation. It is clear that such a level of anxiety as experienced by the subject can be modulated by for example its emotional, psychological, cognitive state, such as for example the state of consciousness of the subject. The LoA is thus an indicator for the level of anxiety as it is experienced of the subject and can thus be used as an indicator of the degree to which the subject experiences anxiety, for example on a cognitive, emotional and/or physical level.

It is thus clear that anxiety, and more specifically anxiety as defined above, includes a cognitive experience and optionally also a physiological experience by the subject. In other words, the level of anxiety as experienced by the subject is a subjective, psychophysiological experience that is influenced to varying degrees by for example biological, psychological, cognitive, physiological, neurophysiological and social factors. The experience that something causes anxiety includes for example cognitive features, such as for example intensity, quality, ... of an anxiety stimulus and/or affective emotional aspects. It should further be noted that in this way anxiety as experienced and purely autonomic physiological stress responses are different, and can for example occur independently of each other.

As explained in further detail below, in the context of this application a Level of Anxiety Index, or LoAI, or Anxiety index, refers to a standardized index indicating the level of anxiety of which the subject is at least cognitively experiencing and optionally physiologically experiencing. In other words, the degree to which the subject experiences anxiety at a cognitive dimension, optionally supplemented by for example an autonomic sensory and/or an emotional level as described in further detail below. This LoAI for example varies systematically with the intensity of anxiety as experienced by the subject, independently of factors not affecting the experience of anxiety. A first limit of the LoAI, for example a lower limit of the LoAI, may for example indicate that the subject experiences no anxiety. A second limit of the LoAI, for example an upper limit of the LoAI, may for example indicate that the subject experiences the strongest/worst possible experience of anxiety.

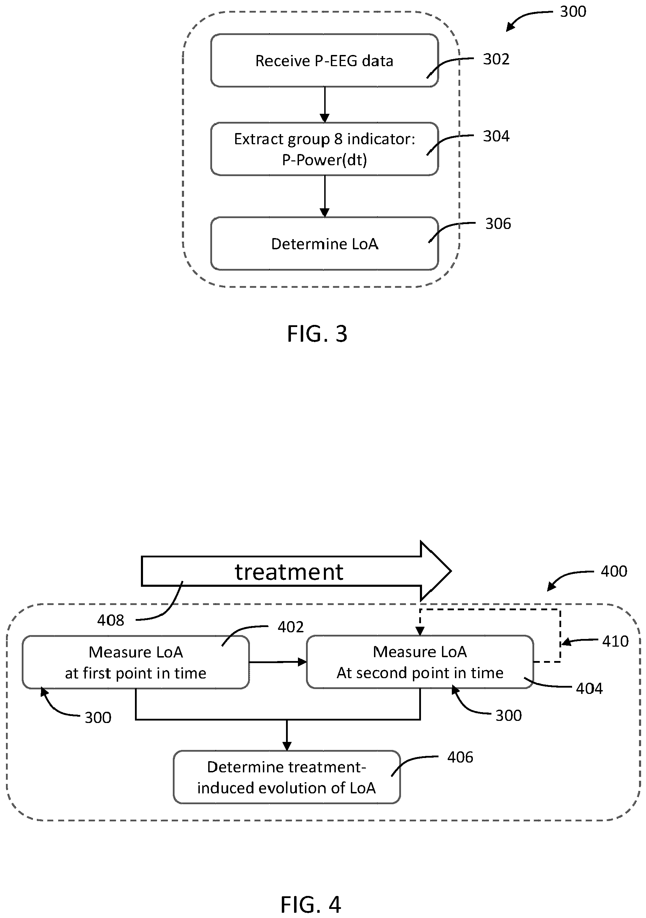

According to the embodiment shown in FIG. 3, such a computer-implemented method for an objective measurement of a level of anxiety 300 as experienced by a subject, for example comprises the steps of receiving EEG data 302, extracting P-power(dt) 304 and determining a level of anxiety, LoA, 306. At step 302 there is received measured data comprising electroencephalogram, EEG, data collected from one or more P-EEG electrodes. At step 304, there is then extracted from the EEG data, a group 8 indicator corresponding to: a power, P-power (dt), associated with a delta-theta frequency band, dt, within a delta-theta frequency range. At step 306 there is then determined, based on said group 8 indicator, a level of anxiety, LoA, which is a value indicative for the level of anxiety as experienced by the subject. It is clear that, according to alternative embodiments, optionally, in addition to said group 8 indicator, further elements, such as other group indicators, measurements, parameters, reference measurements, ... could be taken into account for determining the LoA. It is clear that according to particular embodiments the method could for example comprise additional steps, such as for example after the step of receiving EEG data or any other suitable data, pre-processing the EEG data or other data, subsequently extracting relevant features, thereby allowing the determination of for example group 8 indicators or other suitable group indicators, and subsequently normalization and/or classification, thereby for example determining LoA or an indexed and/or scaled version of LoA, such as for example LoAI as will be explained in further detail below. It is however clear that still further alternative embodiments are possible.

According to the embodiment of the experimental data generated above, it was found that for a population with a mean self-reported anxiety value of 4.92, there was measured a mean P-power(dt) of 0.84 mV$^2$, when a calibrated pain stimulus of value 8 on the pain scale was applied in normal awake state 530. For a population with a mean self-reported anxiety value of 3.16, there was measured a mean P-power (dt) of 0.44 mV$^2$, when a calibrated pain stimulus of value 8 on the pain scale was applied during the hypnotic treatment session 540. As already mentioned above, from the experimental data above, the LoA is correlated to the group 8 indicator, P-power(dt), or in other words an increase in P-power(dt) corresponds to an increase in the LoA, and a decrease in P-power(dt) corresponds to an decrease in the LoA as experienced by the subject. As already mentioned above, it is clear that the P-power(dt) values mentioned above, were calculated from -0.5s to 1.5s from with reference to the stimulus onset, or in other words the power was calculated in a time window of 2s including the stimulus onset, according to an embodiment where there was an external pain and/or anxiety stimulus. As further mentioned above, it is however clear that alternative embodiments are possible, in which alternative suitable time windows are being used, and/or in which the time window is not related to a specific external stimulus, such that for example the power can be continuously calculated based on overlapping time windows.

According to FIG. 4, these embodiments of the computer-implemented measurement of the LoA based on measurement data could be applied to determine and monitor a treatment induced evolution of the LoA. When for example a treatment is applied, such as for example an anxiety reducing treatment or any other suitable treatment, the effect of this treatment on the LoA may be monitored by embodiments of the present method. Such treatments might for example comprise any suitable treatment, such as non-pharmacological treatments, such as for example non-pharmacological anxiety treatments, hypnosis, other evidence-based psychological and/or mind/body interventions, etc. , and/or pharmacological treatments, such as for example pharmacological anxiety treatments, a treatment comprising administration of active pharmacological ingredient, administration of an anxiolytic, and/or any other suitable treatments such as for example medical or paramedical treatment such as surgery, physical therapy, electrical treatment, mechanical treatment, ... , and/or mind/body complementary or alternative medicines such as acupuncture, biofeedback, massage.... In this way FIG. 4 shows an embodiment of a computer-implemented method for measuring and/or monitoring a level of anxiety in a subject before, during and/or after a treatment 400. The embodiment of the method 400 shown in FIG. 4, comprises the steps of:

- measuring the LoA at a first point in time 402, for example before the treatment or at a first point in time during the treatment;
- measuring the LoA at a second point in time 404, for example after the treatment, or at a second point in time during the treatment later than the first point in time; and
- determining the treatment-induced evolution of the LoA 406.

The treatment, and its associated chronology, has been schematically illustrated by means of arrow 408 in the embodiment of FIG. 4, which could for example represent a singular treatment session with a well-defined start and endpoint. However it is clear that alternative embodiments are possible in which the treatment for example has a continuous and/or chronic nature without a well-defined start and endpoint, or in which the treatment has an intermittent nature and comprises a sequence of a plurality of treatment sessions with optional breaks or pauses in between these treatment sessions, or any other suitable type of treatment comprising a chronological nature, for example comprising a sequence of treatment phases, steps, etc.

As shown at step 402 the LoA is measured, for example with the method for measurement of LoA 300 according to the embodiment shown in FIG. 3. This first measurement of LoA could for example be a measurement of the LoA before the start of a treatment session and could, according to some embodiments be considered as a reference, initial, baseline, ... LoA that serves as a predetermined reference level for subsequent measurements of LoA. As further shown in FIG. 4, at step 404, the second, subsequent measurement of LoA, is for example also measured with the method for measurement of LoA 300 shown in FIG. 3. This could for example be a measurement of the LoA during or after the treatment, for example an anxiety reducing treatment. At step 406, then there is determined the treatment-induced evolution of the LoA, based on a comparison of the first LoA and second LoA, measured at steps 402 and 404 respectively. In this way the treatment-induced evolution of the LoA is be able to show a value representative for the effectiveness, intensity, desired effect, etc. of the treatment. If the treatment, for example is an anxiety reducing treatment, then a treatment-induced evolution of the LoA that corresponds to a reduction of the second LoA measured at step 304 as compared to the first LoA measured at step 302, is able to confirm the effectiveness of the treatment. According to some embodiments the value of the treatment-induced evolution of the LoA also provides for an indication of the intensity of the treatment. For example, according to an embodiment, an anxiety reducing treatment reduces a first LoA measurement with a first value at step 402 to second value, that corresponds to a 50% reduction of the first value, at a second measurement at step 404, which thus corresponds to a treatment-induced evolution of the LoA of - 50% or in other words a change in the LoA corresponding to a reduction of 50% with respect to the measurement at step 302 before the treatment. When this treatment is adapted or another treatment is applied, which only causes a treatment induced evolution of the LoA of -25%, it is clear that this latter treatment has an intensity that is lower than the former. When no change is detected with respect to the first LoA measurement, it can be concluded that the treatment-induced evolution of the LoA corresponds to no change to the LoA, or in other words, that the treatment at that point in time is for example not effective in reducing the LoA and might need adaptation or change to another type of treatment. However, according to alternative embodiments, in which for example the treatment comprises the application of pain stimuli and/or anxiety inducing stimuli, such as for example during a surgical treatment, when the LoA as measured before the treatment, does not change, does not increase and/or does not increase above a predetermined threshold, during the treatment, especially during and/or after the application of the pain and/or anxiety inducing stimuli, it is clear that the anxiety treatment, for example in the form of pharmacological and/or non-pharmacological anxiety reducing treatment is performing effectively.

As schematically shown by means of striped line 410, according to an optional embodiment, the measurement of a further LoA at step 404, at a later point in time than at step 402, could be repeated or could be performed continuously, which allows for a repeated and/or continuous monitoring of the treatment-induced evolution of the LoA, or in other words the comparison of two LoA measured at two different points in time. It is clear that alternative embodiments are possible in which any suitable plurality of LoA measurements at different points in time, for example before, during and/or after the treatment, are used in order to determine and/or monitor an evolution, for example a treatment-induced evolution, of the LoA, based on a comparison of these at least two LoA measured at at least two different points in time. It is thus clear that such a treatment induced evolution of the LoA may be indicative of a treatment induced change, if any, to the LoA. However, it is clear that alternative embodiments are possible in which the LoA is measured at at least two different points in time during any suitable time period for determination of any suitable evolution of the measured LoA.

In addition to the group 8 indicator mentioned above with respect to the experiment 500 as shown in FIG. 1 and the application of the results of this experiment in the computer-implemented methods of FIG. 3 and FIG. 4, the LoA can optionally be determined by means of additional indicators, such as for example a group 6, 7, 9, 10 and/or 11 indicator as described in further detail below.

According to such embodiments, during the embodiment of the experiment as shown in FIG.1, in addition to the group 8 indicator, there is for example extracted from the measured data any other suitable group indicator in the time domain, for example by means of a suitable event related potential, or ERP, in response to the calibrated pain and/or anxiety inducing stimulus and/or any other suitable group indicator in the frequency domain, similar as described above.

As will be described in further detail below, and already mentioned above the group 8 indicator is based on measured data comprising EEG data, which can be referred to as a cognitive, electrophysiological signal, and is related to corticoidal signals, and is related to the cognitive dimension of the level of anxiety as experienced by the subject. The group 6, 7, 9, 10 and/or 11 indicators are all related to non-cognitive physiological and/or non-cognitive electrophysiological signals for generating measured data, such as for example cardiovascular data, ECG data, respiratory data, ocular movement data, EOG data, muscular activity data, EMG data, body temperature data, etc. It is thus clear that, a combination of the group 8 indicator for measuring the cognitive dimension of the level of anxiety and optionally, additionally and/or preferably at least one non-cognitive physiological group indicator such as for example group 6, 7, 9, 10 and/or 11 as described in further detail below provides for measuring a combination of this cognitive dimension of anxiety with the autonomic dimension, thereby allowing for measurement of the multi-dimensional aspect of the level of anxiety as experienced by a subject.

According to an exemplary embodiment the measured data may comprise cardiovascular data. Such cardiovascular data may for example be acquired using two or more electrocardiogram (ECG) electrodes placed on the skin using peripheral or precordial placement, or using any other placement where ECG data can be derived. ECG detects electrical activity of the heart. Alternatively, or in addition, cardiovascular data may be acquired using a photoplethysmogram (PPG) sensor placed on the skin. PPG detects blood volume changes vasculature below the skin, from which for example heart rate, heart rate variability, etc. can be derived. Cardiovascular data may alternatively, or in addition be acquired from at least one other suitable electrode, such as for example one or more EEG electrodes, etc, configured to detect a signal from which cardiovascular data, such as heart rate, heart rate variability, ... can be detected and/or extracted. Cardiovascular data may comprise heart rate, heart rate variability, derived metrics, phase-based parameters, frequency-based parameters. However, it is clear that, according to alternative embodiments, ECG measured data, might for example be extracted from any suitable electrodes, such as for example from signals measured by EEG electrodes. In general, and as described in further detail below such cardiovascular data may be received from any suitable cardiovascular data capturing unit.

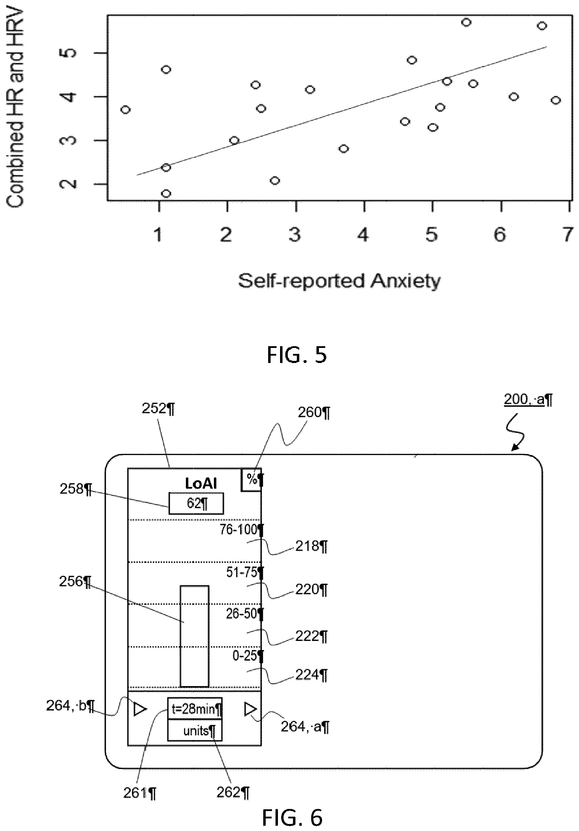

According to such an embodiment, based on the received measured data further comprising the cardiovascular data in addition to the P-EEG data, there can be extracted from the cardiovascular data a group 7 indicator based on a heart rate and/or heart rate variability. Preferably, as will be described in further detail with respect to the embodiments shown in FIG. 5 and/or FIG. 20 - FIG. 23, preferably a specific combination of heart rate and heart rate variability is used to extract the group 7 indicator. Subsequently the LoA is determined based on said group 8 indicator and at least the group 7 indicator.

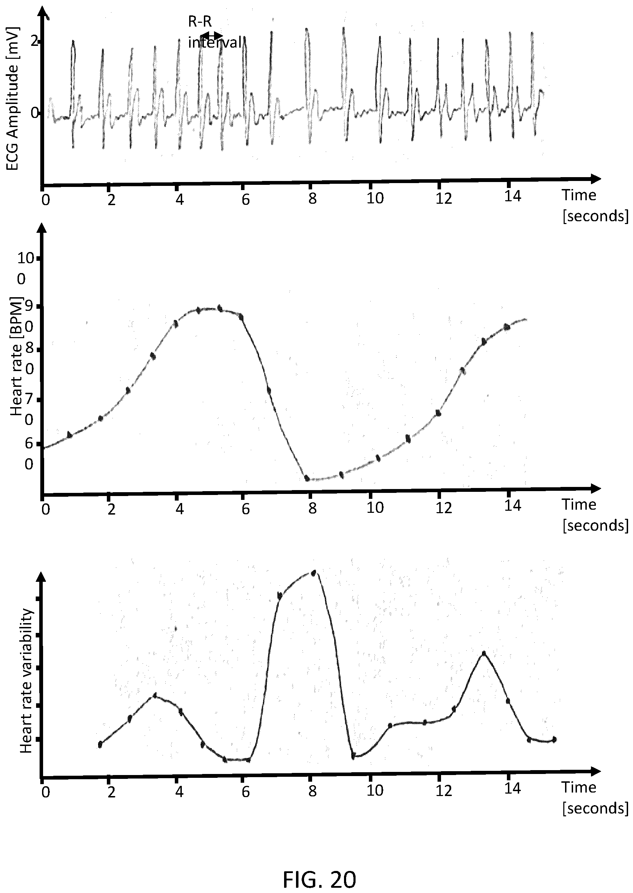

The correlation between a specific combination of heart rate or HR and heart rate variability HRV providing for a group 7 indicator is shown in Fig. 5 for the measurement data according to the embodiment explained above with reference to Fig. 1. As shown, for this specific combination of HR and HRV, as will be explained in further detail below, there was a strong correlation with the self-reported LoA with rs=0.5 and a p-value=0.02. Fig. 20 details an embodiment, in which use is made of ECG data to obtain the HR and HRV. However, it is clearthat alternative embodiments are possible in which the HR and HRV are determined by alternative means, and/or are present in the measured data obtained from the experiment directly. According to the embodiment of Fig. 20, there is shown an example of the amplitude of an ECG signal, subsequently, by means of peak detection in this ECG signal the heart rate can be detected, which is shown as the number of beats per minute or bpm. The variability of the detected heart rate then forms the basis for calculating the heart rate variability. According to the specific embodiment, as shown in Fig. 20, the ECG signal is for example first pre-processed to remove any noise and artefacts. Subsequently, any useful characteristic features of the ECG signal can be extracted such as for example, the P wave, which represents the depolarization of the atria; the QRS complex, which represents the depolarization of the ventricles; and/or the T wave, which represents the repolarization of the ventricles, as known to the man skilled in the art. According to the embodiment shown, the R peaks of each ECG signal are detected, and the R-R interval, i.e. the interval between the R peaks of two consecutive heart-beat ECG signals is determined, and based on this R-R interval the heart rate is calculated. Subsequently based on the R-R interval and/or the derived heart rate, there is performed a time-domain analysis to determine the HRV, based on the difference and/or variability between two adjacent R-R intervals, and/or the heart rate associated with two adjacent heart beats. According to a particular embodiment the HRV could for example be determined based on the standard deviation of the normal R-R interval in a particular time period.

One subject of the embodiment of the experiment above, for example provided a self-reported level of anxiety of 4.1 during the normal awake phase 530, during which a corresponding HR = 58 bpm, which was for example the mean, average, weighted mean, ... of the HR during the normal awake phase. Additionally a HRV = 53 ms was measured, as the standard deviation of the normal R-R interval during the normal awake phase 530. Subsequently during the second phase 540 with the hypnotic treatment session a lower self-reported level of anxiety of 1.1 was provided by the subject and this corresponded to a measured HR = 51bpm and a HRV = 169 ms.

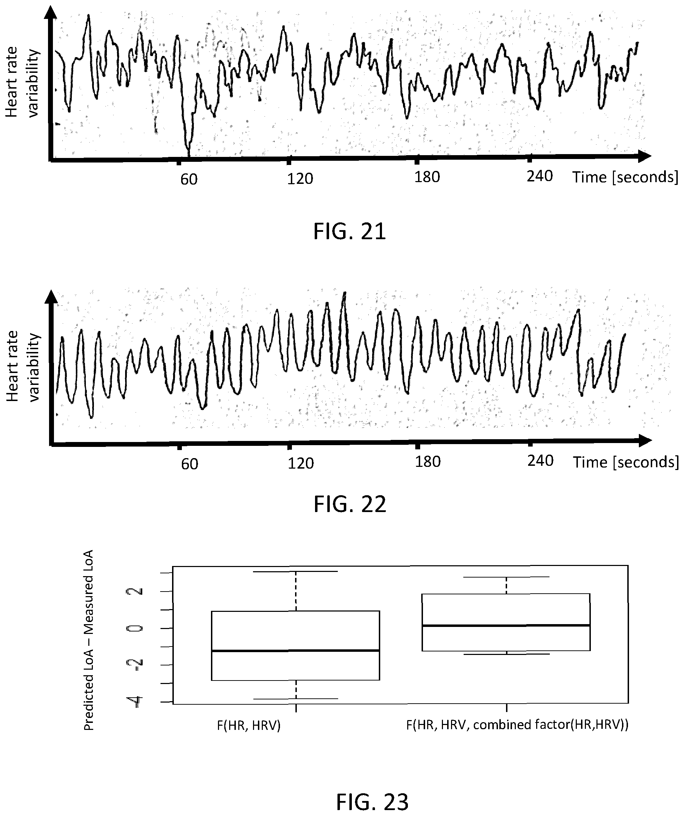

In order to further visualize the effect on the HRV FIG. 21 shows an exemplary heart rate variability for a subject with a higher level of anxiety than in FIG. 22, and FIG. 22 shows a heart rate variability for a subject with a lower level of anxiety than in FIG. 21.

According to a particular advantageous embodiment, in which a combination of the group 8 indicator and the group 7 indicator is used for determining the LoA, there is made use of the following specific formula for computing the LoA:

$$LoA_{s,t} \sim f_4\left(group\ 8\ indicator_{s,t}, \alpha_1 f_1\left(HR_{s,[t',t]}\right) + \alpha_2 f_2\left(HRV_{s,[t',t]}\right) + \alpha_3 f_3\left(HR_{s,[t',t]}, HRV_{s,[t',t]}\right)\right)$$

Where:

- $LoA_{s,t}$ is the anxiety level of subject s at time t;
- $group\ 8\ indicator_{s,t}$ is an embodiment of the group 8 indicator based on P-power(dt) as described above of subject s at time t;
- $\alpha_1 f_1(HR_{s,[t',t]}) + \alpha_2 f_2(HRV_{s,[t',t]}) + \alpha_3 f_3(HR_{s,[t',t]}, HRV_{s,[t',t]})$ is a specific advantageous embodiment of the group 7 indicator.
- $\alpha_i$ is a parameter that is determined in function of for example subject characteristics, population characteristics, population size, ... and that is for example determined using an automated machine learning processes, in which parameters are selected which provide for the best correlation with a predetermined set of measured data and/or reference data, such as for example population based reference data;
- $f_i$ is a mathematical function determined using a similar automated machine learning processes, in which functions are selected that provide for the best correlation with a predetermined set of measured data and/or reference data, such as for example population based reference data;
- $HR_{s,[t',t]}$ is a single value representing the heart rate of subject s during a time period from time t' to time t, e.g. mean value, however alternative embodiments are possible such as an average value, weighted mean, etc.,
- $HRV_{s,[t',t]}$ is a single value representing the HRV of subject s during a time period from time t' to time t, e.g. standard deviation of the normal R-R interval, or any other suitable quantification of the HRV. This specific embodiment of the group 7 indicator is particularly advantageous, as it not only combines the HR and HRV, but also comprises what can be referred to as a combined HR-HRV factor: $\alpha_3 f_3(HR_{s,[t',t]}, HRV_{s,[t',t]})$. To demonstrate the surprising, advantageous effect of this combined HR-HRV, FIG. 23 shows a boxplot of the difference between a predicted LoA and an actually measured LoA based on a model only making use of a function comprising the combination of HR and HRV, in other words $\alpha_1 f_1(HR_{s,[t',t]}) + \alpha_2 f_2(HRV_{s,[t',t]})$, for the calculation of the group 7 indicator, which was labelled "f(HR, HRV)". For comparison, a further boxplot is shown labelled "f(HR, HRV, combined HR-HRV factor)", in which for the calculation of the group 7 indicator use was also made of the combined HR-HRV factor, or in other words $\alpha_1 f_1(HR_{s,[t',t]}) + \alpha_2 f_2(HRV_{s,[t',t]}) + \alpha_3 f_3(HR_{s,[t',t]}, HRV_{s,[t',t]})$. We observe that the prediction accuracy of the model with the combined HR-HRV factor is better as the mean difference = 0.36 and standard deviation = 1.55, which is better than the mean difference = -1.14 and std dev = 2.20 of the model without the combined HR-HRV factor.

Optionally a time-frequency analysis of the HRV could be performed in order to extract from the HRV signal a signal correlated to the breathing rate, which could also be used for determining a group indicator, as will be described in further detail below. It is however clear that alternative embodiments are possible in which the HR and HRV can be extracted from the measured data. As shown in FIG. 21 and FIG. 22, there is a clear effect of the breathing frequency that is recognizable as a sinusoidal pattern with peaks and valleys, of which one cycle corresponds to a breathing cycle, in the HRV signal. It is clear, that when a subject is experiencing a higher level of anxiety as in FIG. 21, the breathing rate is less stable or comprises a higher level of variability then at a lower level of anxiety as shown in FIG. 22.

According to still a further embodiment there is provided a computer-implemented method 300 for measurement of a level of anxiety comprising the further step of receiving measured data further comprising respiratory data. From this respiratory data there can then be extracted a group 6 indicator based on respiration rate and/or a respiration variability and/or a predetermined respiration pattern and/or oxygen saturation. This then allows to determine the LoA based on the group 8 indicator and at least the group 6 indicator, which similar as described above provides for a multi-dimensional measurement of the LoA.

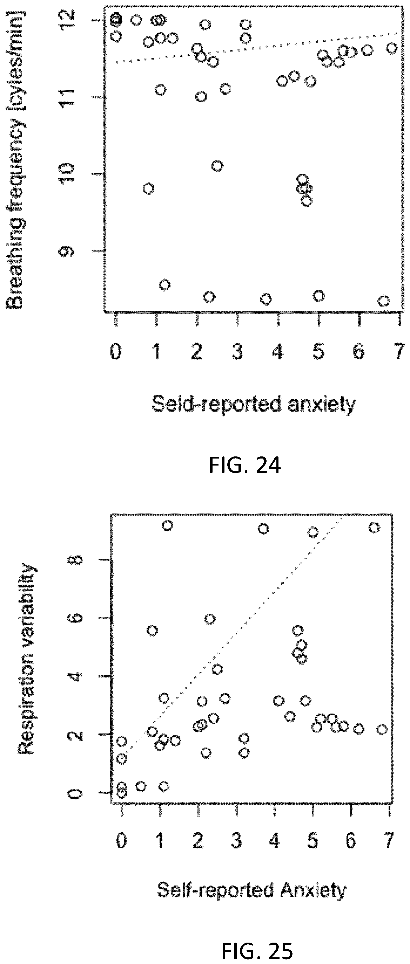

From the measured data from the experiment of Fig. 1, there was found to be a correlation of the respiration rate with the self-reported level of anxiety, with rs= 0.458 and a p-value = 0.002. In other words, as already mentioned above, an increased level of anxiety as experienced by the subject leads to a higher respiration rate. FIG. 24 shows correlation between self-reported anxiety and respiration rate also referred to as breathing frequency for the measured data from the experiment of Fig. 1. It is clear that the respiration rate or the breathing frequency, typically refers to the number of breathing cycles per minute.

Further there was also found a correlation between the respiration rate variability, such as for example the standard deviation of the Inter-cycles interval or ICI, was found to be significantly higher during a period with a higher LoA. There was thus found to be a correlation with the self-reported level of anxiety, with an rs= 0.434, and p-value = 0.005, i.e. an increased level of anxiety leads to a less regular respiration pattern. FIG. 25 shows such correlation between the self-reported level of anxiety and respiration rate variability, also referred to as respiration variability, for the measured data from the experiment of Fig. 1. It is thus clear that the respiration rate variability, such as for example determined as the standard deviation of the ICI, is a measure for the regularity and/or irregularity of successive breathing cycles over a certain period of time.

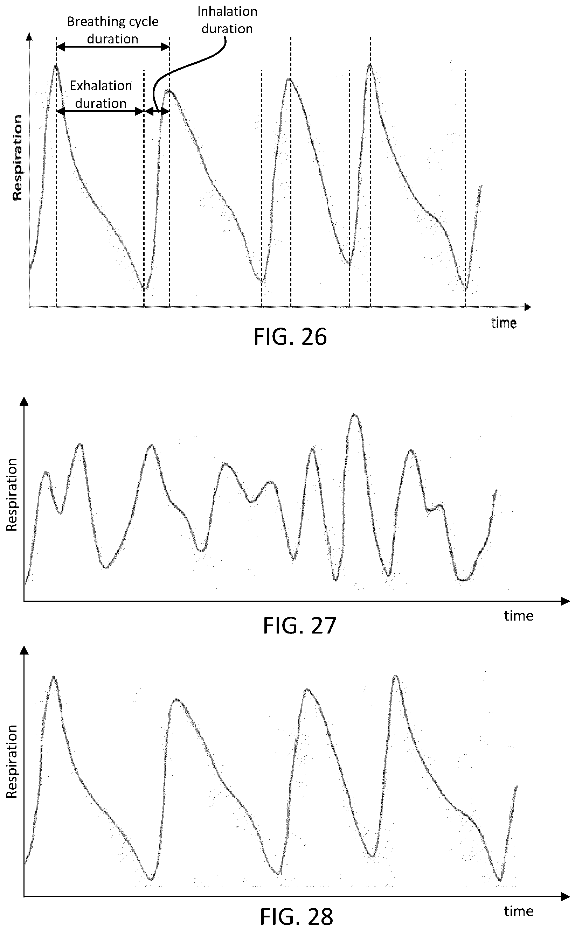

Further FIG. 26 shows an exemplary embodiment of a time domain respiratory data signal, which for example shows flow and/or pressure variations during a few successive breathing cycles, in which a breathing cycle comprises a combination of a successive exhalation and inhalation. FIG. 26 shows such respiratory data during a time period of about 1 minute. As shown, from such respiratory data there could be extracted parameters such as the breathing cycle duration, which is the time period associated with one complete breathing cycle; the exhalation duration, which is the time period associated with the part of the breathing cycle during which the exhalation takes place; the inhalation duration, which is the time period associated with the part of the breathing cycle during which the inhalation takes place; and/or any ratio's and/or any combination of such durations; and/or any other parameters derivable from such time domain respiratory data signal, such as for example the peak to peak amplitude of the signal, the minima and maxima of the signal, and/or variability of such signals, etc. FIG. 27 and FIG. 28 show the respiratory data signals of an exemplary subject that participated in the experiment of FIG. 1. FIG. 27 shows the respiratory data signal of the subject during the normal awake state 530 during which the subject experienced a self-reported level of anxiety = 4.6 . FIG. 28 shows the respiratory data signal of the same subject during the hypnotic treatment session 540 during which the subject experienced a self-reported level of anxiety = 2.5. It is clear from a comparison of FIG. 27 and FIG. 28 that when the level of anxiety is lower, this correlates for example to a lower respiration rate, or in other words a longer breathing cycle duration. Further, as shown a lower level of anxiety also correlates to a more regular breathing cycle, in other words there is less variability regarding the duration and/or amplitude of successive breathing cycles, the duration and/or amplitude of successive inhalation periods, the duration and/or amplitude of successive exhalation periods, etc.. Further, as for example shown, at a lower level of anxiety the exhalation duration is longer than the inhalation duration, while at a higher level of anxiety this is no longer always the case. Any of these parameters, and/or any suitable combination, ratio, etc. thereof thus allows for further embodiments of a suitable group 6 indicator based on respiratory data for determining the LoA. It is however clear that still further alternative embodiments are possible. According to such alternative embodiments, there could for example be taken into account the occurrence of any suitable predetermined respiration pattern, such as for example the occurrence of erratic breathing cycles in which there cannot be made a clear distinction between an exhalation and an inhalation, the occurrence, frequency, and/or duration of pauses between and/or during an inhalation and/or exhalation, etc. It is however clear that alternative embodiments of alternative predetermined respiration patterns are possible, which could be taken into account for embodiments of the group 6 indicator.

According to a particular advantageous embodiment, the LoA is calculated based on the group 8 indicator, in combination with an embodiment the group 6 indicator and the group 7 indicator, or in other words:

$$LoA \sim f\left(group\ 8\ indicator_{s,t}, group\ 7\ indicator, group\ 6\ indicator\right)$$

According to a particular advantageous embodiment, similar as explained above, with respect to the combined HR-HRV factor, preferable there is additionally made use of a combined factor taking into account the influence of the respiration rate or BR on the heart rate and/or the heart rate variability, so similar as explained above such a combined HR-BR factor, such as for example could be included in the formula for calculation of the LoA, such as for example $\alpha_i f_i(HR_{s,[t',t]}, BR_{s,[t',t]})$ and/or $\alpha_i f_i(HRV_{s,[t',t]}, BR_{s,[t',t]})$, for example as part of the group 7 indicator and/or the group 6 indicator.

It is clear that still further alternative embodiments for determining a group 6 indicator from the respiratory data, for example it is possible to take into account data with respect to the volume and/or depth of breathing, which could for example be directly measured, and/or indirectly derived from data relating to oxygen saturation, such as for example by means an SpO2 sensor, or from the amount of belly and/or chest movement during breathing. A person with a higher level of anxiety has been detected to have a shallower breath, with a reduced volume of breathing, then when a subject is experiencing a lower level of anxiety. This is for example also illustrated in the embodiment of FIG. 27 and FIG. 28 as the peak to peak amplitude of the signal of FIG. 28 is larger than that of FIG. 27. It is however clear that still further alternative embodiments are possible, such as for example the detection of a reduced flow, volume and/or pressure of air during an inhalation and/or an exhalation, etc.

According to still a further embodiments there is provided a computer-implemented method 300 for measurement of a level of anxiety comprising the further step of receiving measured data relating to a autonomic physiological parameter for providing a multi-dimensional measurement of the LoA similar as described above and as will be explained in more detail below. According to these embodiments the LoA can be determined based on the group 8 indicator and at least one or more of a group 6, 7, 9, 10 and/or 11 indicator.

According to such an embodiment the received measured data further comprises ocular movement data, and there can be extracted a group 9 indicator based on an ocular movement rate and/or ocular movement amplitude and/or predetermined ocular movement patterns from the ocular movement data. This then allows to determine the LoA based on the group 8 indicator and at least the group 9 indicator, and optionally any other suitable autonomic physiological group indicator.

According to a particular embodiment the ocular movement data was captured as EOG data and the group 9 indicator is based on the detection of saccadic eye movements from the ocular movement data.

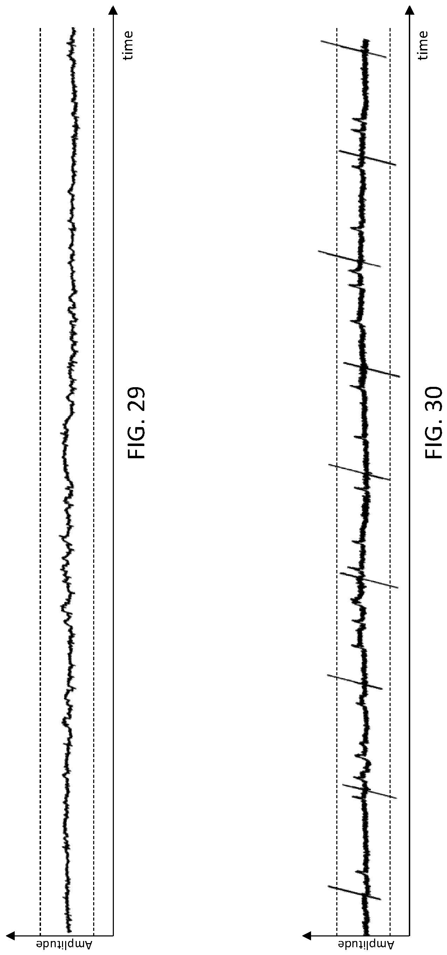

FIG. 29 and FIG. 30 show an embodiment of ocular movement data of a single subject that was captured during the experiment of FIG. 1 in the form of EOG data signals. FIG. 29 shows the EOG data signal of the subject during the hypnotic treatment session 540 during which the subject experienced a self-reported level of anxiety = 2.1. FIG. 30 shows the EOG data signal of the subject during the normal awake state 530 during which the subject experienced a self-reported level of anxiety = 6.8 . The time period of the signal shown is about 30 seconds. In order to determine the frequency of saccadic eye movements, and/or the number of saccadic eye movements during a particular time period, according to the embodiment shown, there can be made use of one or more thresholds, as for example schematically represented by means of the striped lines in FIG. 29 and FIG. 30. According to such an embodiment, for example every time the EOG signal crosses one of these thresholds a saccadic eye movement is detected. As shown in FIG. 29, no saccadic eye movements are detected in the EOG signal for this subject at a self-reported level of anxiety = 2.1 . As shown in FIG. 30 9 saccadic eye movements are detected in the time period of about 30 seconds in the EOG signal for this subject at a self-reported level of anxiety = 6.8 . In other words, the frequency of saccadic eye movements is higher when there is a higher level of anxiety. It is however clear that alternative embodiments are possible in which the number and/or frequency of saccadic eye movements, and or any other suitable parameter related to saccadic eye movements, such as for example the amplitude, direction, predetermined pattern, etc., is extracted from ocular movement data, such as for example EOG data, on which a suitable embodiment of a group 9 indicator can be based for determining the LoA.

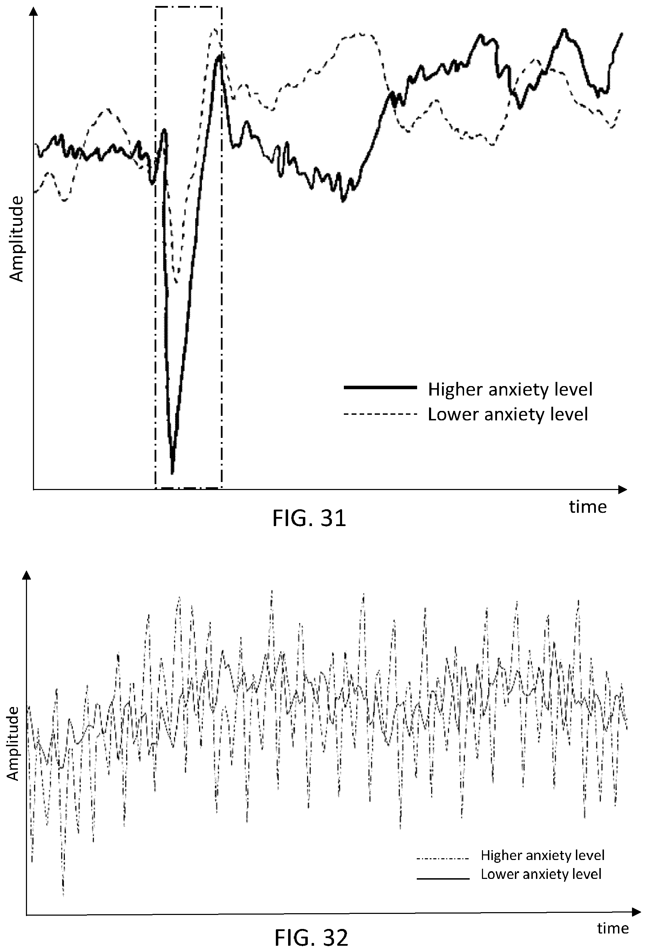

FIG. 31 shows an alternative, exemplary embodiment of time domain ocular movement data, in the form of an EOG data signal, averaged for a plurality of subjects, which is time-locked to a painful stimulus, which serves as an anxiety inducing stimulus. In other words, there is shown an event-related potential or ERP for the EOG data signal which is time-locked to the painful stimuli during the experiment of FIG. 1. According to the exemplary embodiment shown, the time-domain signal can for example be analysed, during a specific time window, as schematically shown by means of the dot striped box, which is for example approximately 2 seconds, during which for example a peak amplitude, a peak to peak amplitude, and/or any other suitable amplitude based parameter with respect to the ocular movement data signal can be determined. The full line graph shows the averaged EOG signal of all subjects during the experiment of FIG. 1 during the normal awake state 530, and the striped graph shows the averaged EOG signal for all subjects during the hypnotic treatment session 540. It is clear that in the time window, the peak to peak amplitude of the eye movement data signal is larger for the signal with a higher level of anxiety. Further, according to this specific embodiment, specifically the negative peak amplitude of the EOG signal, or in other words the minimum value of the EOG signal in the time window shows a clear difference at different levels of anxiety. For the experiment of FIG. 1 the average value of the amplitude of the EOG data signal during the time window was for example -17.8 mV during the normal awake state 530 which corresponds to a higher level of anxiety, while it was only -7.6 mV during the hypnotic treatment session 540. It is however clear that alternative embodiments are possible in which, for example based on the amplitude of the ocular movement data, there can be determined a suitable embodiment of a group 9 indicator for determining the LoA. It is however clear that alternative embodiments are possible in which the amplitude of the EOG data, and or any other suitable parameter related to ocular movement is extracted from ocular movement data, such as for example EOG data, on which a suitable embodiment of a group 9 indicator can be based for determining the LoA, such as for example ocular movement rate, ocular movement amplitude, predetermined ocular movement patterns, detection of saccadic eye movements, one or more characteristics of saccadic eye movement patterns, such as for example frequency, amplitude, direction and/or predetermined saccadic eye movement patterns.

The EOG data could be captured by a suitable sensor for electrooculography, which is a technique for measuring the corneo-retinal standing potential that exists between the front and the back of the human eye. The resulting signal is called the electrooculogram or EOG. An EOG allows to record eye movements.

There are four basic types of eye movements: saccadic eye movements, smooth pursuit eye movements, vergence eye movements, and vestibulo-ocular eye movements. Saccadic eye movements are rapid, ballistic movements of the eyes that abruptly change the point of fixation. It has been found that the frequency and/or number in predetermined time frame of saccadic eye movements increases when a higher level of anxiety is experienced by a subject.

According to such a further embodiment the received measured data further comprises muscular activity data, such as for example electromyography, EMG, data from which a group 10 indicator based on muscular activity, such as for example electromyographic, EMG, activity can be extracted, which allows to determine the LoA based on said group 8 indicator and at least the group 10 indicator, and optionally any other suitable group indicator.

FIG. 32 shows an embodiment of muscular activity data of a single subject that was captured during the experiment of FIG. 1 in the form of EMG data signals, of for example mastoid muscles or any other suitable muscles; in the time domain. The signal shown in dot striped line is an EMG signal captured for this subject during the normal awake state 530, during which the subject reported a level of anxiety = 6.8 . The signal shown in the full line is an EMG signal captured for this subject during the hypnotic treatment session 540, during which the subject reported a level of anxiety = 2.1 . In other words, the EMG signal in the dot striped line is associated with a higher level of anxiety, while the EMG signal in the full line is associated with a lower level of anxiety. It is clear from the embodiment shown, that for example the peak to peak amplitude, of the EMG signal is higher at higher levels of anxiety, or in other words, there is more muscular activity, and/or a higher level of muscular contraction when the subject experiences higher levels of anxiety. It is thus clear that, for example based on such differences in the amplitude of the EMG signal, a suitable embodiment of the group 10 indicator could be determined for determining the LoA. It is however clear that further embodiments are possible in which a group 10 indicator is based on any suitable parameters of muscular activity data, for example extracted from an EMG signal, such as for example the amplitude, frequency, variability, predetermined patterns, etc.

According to such a further embodiment the received measured data further comprises body temperature data from which a group 11 indicator based on body temperature can be extracted so that the LoA based on said group 8 indicator and at least the group 11 indicator or any other suitable indicator.

Similar as described above, according to a preferred embodiment, the method for measuring the LoA 300 comprises the step of receiving the measured data of the subject comprising the electroencephalogram, EEG, data collected from one or more of the following EEG electrodes:

- a frontal (F) EEG electrode configured for collection of F-EEG electrode data from the scalp anatomical region corresponding to a frontal lobe of the subject,
- a parietal (P) EEG electrode configured for collection of P-EEG electrode data from the scalp anatomical region corresponding to a parietal lobe of the subject,
- a central (C) EEG electrode configured for collection of C-EEG electrode data from the scalp anatomical region corresponding to a precentral and postcentral gyrus of the subject.

Such EEG data is related to brain waves generated by parts of the brain close to the scalp, including the cortex, and therefor are related to the level of anxiety as cognitively experienced and optionally physiologically experienced by the subject.

Electroencephalography (EEG) is a technique well known in the art for recording electrical activity of the brain. EEG Electrodes placed along the scalp at certain locations measure voltage fluctuations resulting from electrical impulses within the neurons of the brain, mainly in the region of the brain close to the scalp, including the cortex. EEG measurement data reflects how different neurons/ populations of neurons in the brain networks communicate with each other by means of electrical impulses. Electroencephalography produces an electroencephalogram. The electroencephalogram, which thus forms the EEG data, may be refined to a particular electrode location, such as the frontal, parietal or central electrodes mentioned above. From the EEG data there may for example be extracted a frequency-based parameter and/or a phase-based parameter, and/or amplitude-based parameter. Frequency-based parameters may for example be obtained by a frequency-based analysis of EEG measurement data. Such a frequency based analysis of EEG measurement data may for example be used to measure the power contained in a specific signal frequency band, where power refers to a square of amplitude of the frequency domain signals typically within a specified in a frequency band or range, such as for example a frequency band spanning or within a delta-theta frequency range.

The "delta-theta" (dt) frequency band refers to a band of frequencies in a frequency range encompassing both delta and theta brain waves. The delta-theta (dt) frequency range is typically greater than or equal to 0 Hz and equal to or less than 8 Hz. The theta brain waves are preferably slow theta brain wave, for example greater than 3 Hz and equal to or less than 6 Hz. The width of the delta-theta (dt) frequency band may be at least 2 Hz. Most preferably the delta-theta (dt) frequency band is greater than or equal to 1 Hz and less than or equal to 6 Hz. In other words, the delta-theta (dt) frequency band comprises and preferably consists of a combination of at least part of the delta frequency band and at least part of the theta frequency band.

The EEG data may be acquired with one or more EEG electrodes placed on the scalp in the anatomical regions corresponding to the position of the frontal lobe, the parietal lobe and/or both precentral and postcentral gyrus.

A frontal EEG electrode or F-EEG electrode is located on the scalp anatomical region corresponding to the frontal lobe. The F-EEG electrode is configured for attachment to or contact with the scalp anatomical region corresponding to the frontal lobe. The F-EEG electrode is configured to record F-EEG electrode data. The frontal lobe refers to the anatomical part of the brain. The region of the frontal lobe may be along the sagittal plane, more preferable in the middle of the scalp anatomical region corresponding to the frontal lobe, along the midsagittal plane

A parietal EEG electrode or P-EEG electrode is located on the scalp anatomical region corresponding to the parietal lobe. The P-EEG electrode is configured for attachment to or contact with the scalp anatomical region corresponding to the parietal lobe. The P-EEG electrode records P-EEG electrode data. The parietal lobe refers to the anatomical part of the brain. The region of the parietal lobe may be along the sagittal plane, more preferable in the middle of the scalp anatomical region corresponding to the parietal lobe, along the midsagittal plane

A central EEG electrode or C-EEG electrode is located on the scalp anatomical region corresponding to the precentral and postcentral gyrus. The C-EEG electrode is configured for attachment to or contact with the scalp anatomical region corresponding to the precentral and postcentral gyrus. The C-EEG electrode records C-EEG electrode data. The precentral and postcentral gyrus refers to the anatomical part of the brain. The region of the precentral and postcentral gyrus may be along the sagittal plane, more preferable in the middle of the scalp anatomical region corresponding to the precentral and postcentral gyrus, along the midsagittal plane.

It is clear that alternative embodiments are possible in which in one or more EEG electrodes are arranged at the above positions, and or additional suitable scalp anatomical regions. As a minimum one localized EEG electrode may be used, such as for example positioned at the scalp anatomical region of the parietal lobe. However, it is understood that a second electrode, or ground electrode may be employed for common mode rejection, i.e. to dissociate relevant information from noise; this can be placed anywhere on the scalp, for instance in another region of the scalp, in a bilateral position. A third electrode, or reference electrode, may be employed to compute voltage differences. This third electrode can be placed anywhere on the scalp, for instance at an electrically neutral location.

It is thus clear that the measured data may comprise one or more measured data components, such as for example EEG data, which is a measured data component, such as for example also EMG data, ECG data, EOG data, ....

In addition to the measured data, the embodiments of the experiment 500, method for measurement of the LoA 300, etc. may in addition to measured data optionally also take into account observational data and/or self-reported data. In other words, these methods may make use of response data that may comprise measured data and optionally observational data and/or self-reported data. The response data may comprise measured data and/or observational data and/or self-reported data. The response data contains one or more data components, each component being derived from a single measurement (e.g. EEG, EMG, EDA, ECG, EOG), single observation (e.g. movement, skin colour), or single self-reported event such as for example a self-reported level of anxiety as mentioned above.

It is clear that the measured data is data obtained from the subject using one or more devices such as an electrode and/or sensor, such as for example a transducer, camera, motion sensor, ... disposed in relation to the subject. Observational data is then data obtained from a non-self, or hetero, observation of the subject. Self-reported data is data or information reported by the subject, for example before, during or after the measurement of the LoA and/or the treatment.

According to particular embodiments the response data may comprise electrical activity data. Electrical activity data is measured data. Electrical activity data is any data derived from a measurement obtained by an electrical electrode. The electrode is typically placed on the skin. Usually the data is captured by at least 2 electrodes (e.g. 2, 3, 4, 5 or more). Examples of electrode-captured data include electroencephalogram (EEG) data, electromyography (EMG) data, electrodermal activity (EDA) data, galvanic skin response (GSR), electrocardiogram (ECG) data, electrooculogram (EOG) data. It is preferred that the response data comprises at least EEG data. Any electrical data component (e.g. EEG data) may or may not be processed prior to being used as measured data and/or response data.

According to particular embodiments the response data may comprise physiological data other than such electrical activity data. Such specific physiological data is measured data. Such specific physiological data is data derived from a measurement on the subject obtained by a device usually containing a transducer, camera, motion sensor. Such specific physiological data excludes the above-mentioned electrical activity data. Examples of such specific physiological data include pulse rate data, cardiovascular data (e.g. heart rate, heart rate variability data), blood pressure data, respiratory (rate) data (e.g. respiratory rate, respiratory rate variability data), brain oxygenation data, blood O2 saturation data, regional and/or central blood O2 saturation data, body temperature data, photoplethysmogram (PPG) data. Blood oxygen saturation may be, for instance SpO2, SvO2, and the like. Any physiological data component (e.g. pulse rate data) may or may not be processed prior to being used as response data. It is clear that, according to some embodiments, such specific physiological data could be derived from electrical activity data, such as for example cardiovascular data could for example be derived from ECG data, etc.

According to exemplary embodiments in the response data further comprises ECG and/or cardiovascular data and/or respiratory data and/or muscular activity data and/or EOG data and/or body temperature data, from which the LoA can be determined.

According to particular embodiments the response data may comprise body temperature data. Body temperature data is measured data, typically measured by means of a temperature sensor, for example a temperature sensor contacting the body, however alternative embodiments are possible such as for example non-contact type sensors and/or camera's configured to measure the body temperature, for example based on infrared measurements.

According to particular embodiments the response data may comprise motion-tracking data. Motion-tracking data is measured data. The motion tracking data is data derived from a movement of the body or of a part of the body. Typically, motion tracking data is measured by one or more motion sensors (e.g. a 2- or 3-axis accelerometer, gyroscope, one or more cameras). The motion-tracking data may comprise body one or more of body tracking data (e.g. head, limb (arms legs hands), bodily shaking) and/or eye tracking data. The motion may be spontaneous and/or as a result of a stimulation.

According to particular embodiments the response data may comprise facial expression data. Facial-expression data is measured data. The facial expression data is data relating an emotion, such as for example anxiety. Typically, facial expression data is measured by one or more cameras directed at the face (or EMG sensors).

The ocular movement data also referred to as eye tracking measurement data may include one or more of pupil dilation, eye fixation, blink rate or frequency, eye-EOG, eye movement, blink closing duration, blink flurries, eye closure rate, eyelid distance change (with respect to the normal eyelid distance). Pupil dilation can give information about the activity in the coeruleus, the main site of norepinephrine (stress hormones) synthesis in the brain, as well as the state of sleepiness/ alertness.

Electrooculogram (EOG) measurement data may be acquired using two or more EOG electrodes placed on the skin around the eye (e.g. above and below, or to the left and right of the eye). EOG records eye movements based on the corneo-retinal standing potential that exists between the front and the back of the human eye. EOG data may comprise eye movements/fixation, eye blinks, synchronization of both eye's movements.

According to some embodiments cardiovascular data and/or respiratory data could also be derived from motion tracking data and/or data generated by one or more cameras directed at the face.

According to particular embodiments the response data may comprise observational data. The observational data is data observed about the subject by another person. The observational data is data observed by the user (e.g. care provider such as a physician, or medical assistant, or non-medical assistant (e.g. friend, relative, helper)), or by a stakeholder associated with the user (e.g. hospital), or obtained from a database (e.g. medical records). The observed data may comprise one or more observed data components (e.g. a subject's movements is an observed data component). Observational data may include one or more of subject's movements/ lack of movement; procedural events; clinical observation (skin color, groaning or verbalization of discomfort...); age, type of surgery, ethnic origin, language; dosages of sedation drugs.

According to particular embodiments the response data may comprise self-reported data. The self-reported data is data reported by the subject. The self-reported data may for example be provided during a treatment session. Preferably, the self-reported data is provided before, during and/or after the treatment session. The self-reported data may comprise one or more self-reported data components (e.g. level of anxiety during measurement, or during treatment is a self-reported data component). Self-reported data may include a self-reported level of anxiety for example before, during and/or after treatment, estimated duration of the procedure, recall of the events during the session, ....

According to embodiments the muscular activity data for example comprises EMG measured data, EMG data, which may be acquired using two or more EMG electrodes placed on the skin, preferably on limbs or face; or derived from another sensor like EEG. It represents electrical activity data from the subject muscle tissue. It may comprise a frequency-based parameter and/or a phase-based parameter and/or an amplitude-based parameter. However, it is clear that according to alternative embodiments EMG measured data, might for example be extracted from any suitable electrodes, such as for example from signals measured by EEG electrodes.

According to further embodiments the following measured data may optionally and/or additionally be captured and processed during alternative embodiments of the methods described above and/or below.

Cardiovascular data may be acquired using two or more electrocardiogram (ECG) electrodes placed on the skin using peripheral or precordial placement, or using any other placement where ECG data can be derived. ECG detects electrical activity of the heart. Alternatively, or in addition, cardiovascular data may be acquired using a photoplethysmogram (PPG) sensor placed on the skin. PPG detects blood volume changes vasculature below the skin. Cardiovascular data may comprise heart rate, heart rate variability, derived metrics, phase-based parameters, frequency-based parameters. However, it is clear that according to alternative embodiments ECG measured data, might for example be extracted from any suitable electrodes, such as for example from signals measured by EEG electrodes.

Respiratory measurement data may be acquired using one or more force/movement transducer (e.g. chest band) or air pressure sensor, or derived from a PPG sensor or from ECG electrodes placed at location where respiratory rate is detectable. The respiratory data may comprise one or more of respiration rate, respiration rate variability (also known as respiration variability), inhalation pressure, exhalation pressure, respiratory rhythm, respiratory depth, respiratory pattern and derived indexes. Changes of respiration patterns may for example also indicate changes to the LoA. Respiratory data may be known herein as respiratory rate data. However, it is clear that according to alternative embodiments Respiratory measurement data, might for example be extracted from any suitable sensors, such as for example from signals measured by a 2- or 3-axis accelerometer or a gyroscope.

The response data according to a particular embodiment may comprise measured data that comprises EEG data, and optionally muscular activity data, cardiovascular data, and ocular movement data, ... , observational data that comprises patient's observed movement or lack of movement and dosages of received medication and/or self-reported data that comprises self-reported level of anxiety ratings, .... Preferably the response data comprises EEG data. Preferably the self-reported data (if any) comprises ratings for a level of anxiety.

According to a particular embodiment a value of P-power (dt) compared with a reference value is further indicative of the LoA experienced by a subject. For example, a value of said P-power (dt) compared with a reference value is further indicative of the LoA experienced by a subject. According to such an embodiment an increase of P-power (dt) compared with a respective reference value(s) is indicative of an increased LoA and vice versa.

As will be explained in further detail below such reference values, or reference data can be used to scale and/or index the measured data, such as for example P-power(dt) or any other measured data and/or the parameters determined from it, such as for example the LoA. According to such embodiments reference data comprising measured data of a subject and/or a population correlated to at least one predetermined reference LoA is received. This could for example be reference data generated by the subject itself, such as a process similar as the calibration phase or the normal awake state of the experiment, for example prior to a treatment session. According to alternative embodiments the reference data and the correlations could result from separate and distinct subject or population-based reference measurements. There can then be determined at least one correlation of at least one predetermined reference LoA and at least one group indicator extracted from the reference data. In this way, similar as described above, for example one or more particular values of the group 8 indicator P-power(dt) can be correlated with one or more corresponding particular reference values for LoA. These correlations then allow to scale and/or index any subsequently measurements of such a group indicator, such as for example the group 8 indicator P-power(dt). It is clear that similarly for other group indicators there can be scaled and/or indexed a subsequently measured LoA with respect to the at least one predetermined reference LoA by means of said at least one correlation.

Similar as explained above with reference to the group 8 indicator, also for any of the other group indicators, such as the group 6, 7, 9, 10, and/or 11 indicator there can be made use of reference data to scale and/or index the measured data for determining the LoA. It is clear that similar as described above with respect to group 8 indicator, also for any the group 6, 7, 9, 10, and/or 11 indicators, and/or any suitable combination of group indicators and/or other group indicators, there can be scaled and/or indexed a subsequently measured LoA with respect to the at least one predetermined reference LoA by means of said at least one correlation, which could be subject based or population based.

Described herein are one or more indexes, which are standardized indexes indicating the level of anxiety as experienced by the subject. Typically, an index is standardized or normalized as compared to a reference. As mentioned above such a reference could for example be subject-based or population based.

Indexes described herein include a level of anxiety index (LoAI).

A level of anxiety index (LoAI) is a standardized index of the level of anxiety (LoA) indicating the level of anxiety as experienced by the subject. Such an index also allows to determine how much the LoA has been modified, for example by a treatment session, in particular by a pharmacological, and/or non-pharmacological treatment session as compared to a reference state.

An index, for example one or more of LoAI, may have a scale with a first and a second limit. The first limit may represent a lowest level of anxiety, or alternatively a lowest level of anxiety associated with no anxiety and the second limit may represent the highest level of anxiety when compared to a reference state. The first limit may represent an initial state or a reference state of the subject and/or population. The first limit may represent a normal level of anxiety and the second limit may represent a highest level of anxiety. The first limit may represent a normal level of anxiety, and the second limit may represent a significantly increased level of anxiety. The first and second limits may be numerically represented e.g. 0 to 20, 20 to 0, 0 to 60, 60 to 0, 0 to 100, 100 to 0, 0 to 1, 1 to 0 respectively. The second limit may be higher than the first limit, or the first limit may be higher than the second limit. The choice of a higher number for the second limit may depend on the subject or user perspective. An anesthetist may have a preference that the first limit is higher (e.g. 1, 60, 100, 100%) than the second limit (e.g. 0, 0%).

A subject with an index, for example a LoAI which remains close to the first limit, for instance, can be safely approached for further treatments, for example as long as the level of anxiety remains sufficiently low.

The index may have a scale between the first and second limits; the scale may be linear, logarithmic, or other. The scale may be continuous, categorical, discrete, percentage, ratio.

The index, e.g. LoAI, may be indicative of a number of different levels of anxiety or different LoA, for the subject. The number of levels may be any, for instance 3 to 10, preferably 4 to 8 levels. There may be 4, 6, or 8 levels. The levels may be divided within the first and second limits. The division may be even (linear), logarithmic, or according to another scheme. The lowest level (e.g. 1st level) may correlate with or contain the first limit, the highest level (e.g. 4th level) may correlate with or contain the second limit. When an index is a value between 100 (first limit) and 0 (second limit) and the number of levels is 4, the 4 levels may be 100-76 (1st level), 75 to 51 (2nd level), 50 to 26 (3rd level), 25 to 0 (4th level). It is appreciated that the end points of the levels may allow a continuous numerical scale (not interrupted) into the next level. When the index is a value between 100 (first limit) and 0 (second limit) and the number of levels is 8, the 8 levels may be 100-87.5 (1st level), 87.5 to 75 (2nd level), 75 to 62.5 (3rd level), 62.5 to 50 (4th level), 50 to 37.5 (5th level), 37.5 to 25 (6th level), 25 to 12.5 (7th level), 12.5 to 0 to (8th level). The skilled person would be able to determine the extent of the scale (e.g. 100 to 0, or 60 to 0), the number of levels within the scale (e.g. 4, 6 or 8), the type of scale (e.g. linear, logarithmic) and the boundaries between the levels according to the situation.

The index may be a subject-based index in which state of each subject is compared to a reference state of the subject itself or of a group of subjects, or one or more particular populations. The index may be a population-based index in which the state of each subject is compared to a population-based reference. It is clear that alternatively, the index may also be scaled according to an absolute scale. It is clear that any suitable combination of relative, absolute, individual and/or population-based could be used to scale the index.

With respect to the subject-based index, a reference state e.g. a reference value for LoA, is for example measured for the subject before the treatment session. During or after the treatment session, based on response data comprising measured data, real-time values for one or more of LoA is measured for the subject. During the treatment session, based on a comparison between the reference value and the real-time LoA values, real-time values for one or more of LoAI is calculated.

With respect to the population-based index, a reference state e.g. a reference value for one or more of LoA is for example drawn from a database. The database for example comprises reference values that are based on population studies. The reference value may vary according to certain factors include age, gender, ethnicity, intervention characteristics hence, the reference value may be adapted according to the subject and/or the user. During, or after the treatment session, based on response data comprising measured data, real-time values for one or more of LoA is measured for the subject. During, or after the treatment session, based on a comparison between the database comprising the population-based reference value and the real-time LoA values, real time values for one or more of LoAI is calculated.

It is clear that, alternatively, for example the LoA could be scaled according to a subject-based or population-based absolute scale, such that no further reference measurement is required, and that for example measurement of LoA to determine real-time values could be performed without the need for any treatment session. In other words, with a subject-based or population-based absolute scale it is possible to measure LoA and/or LoAI in any suitable context.

The response data may be transformed into an index e.g. into a LoAI using an evaluation protocol. The evaluation protocol may comprise use of one or more of a mathematical (e.g. statistical) model, trained machine-leaning model, mathematical index, reference data. The evaluation protocol takes as input the response data, and outputs the index.

The evaluation protocol may include a step of extracting from the response data of the group 8 indicator and optionally one or more of the Group 6, 7, 9, 10 and/or 11 indicator.

The measured data may comprise one or more data components. A data component is attributed to a single measurement data, for example EEG data, ECG data, ... , a single observation data, for example movement, skin colour, ... , or a single self-reported event data, for example a self-reported level of anxiety, ....

The evaluation protocol may weight the data components of the response data equally or differently. The weighting given to a data component depends, amongst other things, on relevance and precision of the component. For instance, the evaluation protocol may give EEG data a higher rating, reflecting its high relevance and precision. The evaluation protocol may use data components which have less relevance and precision, that are still indicative enough for the situation. In some situations, the response data may comprise a smaller number of data components that have high relevance and precision that are indicative enough for the situation. In some situations, the response data may comprise a larger number of data components that have lower relevance and precision that are indicative enough for the situation. It is clear that various embodiments are possible in which for example according to some embodiments the weighting given to the different group indicators mentioned throughout this description for calculation of the LoA could be adapted in order to adjust and/or fine-tune the calculation of the LoA depending on for example the desired weight and/or effect of the different dimensions of the LoA. According to some embodiments the weighting could be configured to provide a greater weight to the group 8 indicator, when the LoA is measured in a context where the cognitive dimension of LoA outweighs that of the other dimensions of LoA. However it is clear that according to alternative embodiments the group 8 indicator could be provided with a lower weight, and/or be outweighed by other group indicators in a context in which the combination of the cognitive dimension of the LoA with other dimensions of the LoA is more important.

The evaluation protocol may be refined as more subjects are evaluated using the method. A method of refining the evaluation protocol may comprise:

- receiving response data of a subject, for example representing a subject's response to a treatment session,
- receiving independently measured data of LoA(I),
- using the response data and independently measured data to refine the evaluation protocol.

A method of refining the evaluation protocol may comprise:

- Receiving measured response data of a subject, for example representing a subject's response to a treatment session,
- Receiving self-reported and/or observational measure of LoA(I);
- Receiving independently measured data of LoA(I);
- Using the response data, the self-reported and/or observational data, and the independently measured data to refine the evaluation protocol.

The evaluation protocol may additionally or alternatively be refined using analytics. The data collected before, during and/or after one or more previous measurements, and/or one or more previous treatment sessions may contribute a refinement of the evaluation protocol.

As the evaluation protocol is created and/or refined, it may be apparent that one or more data components of the response data become redundant in measuring, or in other words determining, and/or monitoring the level of Anxiety, or for example the modification of the Level of Anxiety by means of a pharmacological and/or non-pharmacological treatment, for example involving an anxiety treatment, a treatment involving the modification of the state of consciousness of a subject, etc.. According to a preferred embodiment at least one, preferably all, of the measured data, observation data and response data are used to create and/or refine the evaluation protocol. According to a preferred embodiment only measured data is used in the method for measuring the LoA.

An embodiment of an evaluation protocol for determining a LoAI is set out below. Generally speaking, it may for example comprise 5 principal steps:

Step 1: receive and digitize response data comprising EEG data, for example during a treatment session. The EEG data could for example be provided from a P-EEG electrode, and optionally one or more of C-EEG and F-EEG electrodes or any other suitable EEG electrodes and optionally from one or more ECG electrodes. According to still further embodiments, optionally there could be provided suitable capturing units, where applicable comprising suitable sensors and/or electrodes to capture one or more of the following: cardiovascular data, respiratory data, ocular movement data data, muscular activity data, body temperature data, etc.

Step 2: generate:
Group 8 indicators P-power(dt) from the digitized response data, and optionally Group 6, 7, 9, 10 and/or 11 indicators.
Step 3: generate one or more indicator values, which means values for any of the group indicators, such as for example group 8 indicator, group 6, 7, 9, 10, 11 indicator, or any other suitable group indicators, from step 2), using for instance, integrative analysis.
Step 4: comparing the value of Step 3) with reference values, for example a population-based index or subject-based index such as mentioned above.
Step 5: obtain from Step 4) a LoAI index e.g. a number of a scale 0 to 20, or a word, or category. It is clear, as explained in further detail in this description that such a LoAI index according to some embodiments could be provided by means of a suitable graphical visualization.

In step 2, the digitized response data may undergo a pre-processing protocol including one or more of re-referencing to average reference, bandpass and/or notch filtering to exclude low and high frequency artifacts, signal segmentation into so called epochs (= sets of x seconds time windows), artifact rejection (ocular, muscular, ...), and baseline adjustment. For time domain analysis of EEG, ECG, EMG, EOG, body temperature, ... signals, there is typically peak detection to for example determine the time period between peaks, peak amplitude, latency (in form of a time series if multiple epochs), and mathematical transformations performed (e.g.: mean or change variable if multiple epochs. The time window for measurement could for example be in the range of 0.01s to 10s, such as for example in the range of 0.1s to 5s.

For a frequency domain analysis of for example EEG signals: there is typically a time to frequency transform (e.g. Fourier transform), relevant frequency bands (e.g. delta-theta (dt)) are extracted, amplitude/power and phase for each frequency band (and each epoch) are determined, and mathematical transformations performed (e.g.: mean if multiple epochs).

For signals analysed in the time domain, peaks can be detected, time interval between peaks, mathematical transformations performed (e.g.: mean if multiple epochs).

From this, one obtains at least one of the following features for at least one electrode: delta-theta frequency band related amplitude/power, delta-theta frequency band related phase, ECG R-R peak interval, .... There may be a normalization step for instance to correct for the time of intervention to ensure that every subject contributes similarly.

In step 3, one or more group indicator values is generated using integrative analysis, for instance, using the equation:

$$LoA_{s,t} = \sum_{i=1}^{n} (\alpha_i \, f_i(F_{i,s,[t',t]}))$$

Where $\alpha_i$ is an optional parameter that evolves with the population size and characteristics, and may be determined using learning processes based on response data and/or independently measured data of the LoA, $f_i$ is a function determined using learning processes based on response data and/or independently measured data of the LoA, $F_{i,s,[t',t]}$ is the value(s) of the feature obtained from point 2 from time t' to time t (t'<t) and for the subjects. The above-mentioned [t' - t] interval means that the value of LoA at time t may be computed based on features extracted from several $F_{i,s,t}$ values. As example, taking the mean and the standard deviation from the past x milli-seconds leads to a more sensitive and more robust determination of LoA. It is clear that the [t' - t] interval thus refers to the time window mentioned above.

In step 4, there is a comparison of values with reference values

If the index is a subject-based index (each subject is its own control),

$$\Delta_r LoA_{s,t} = S \left( \beta \left( LoA_{s,p}^* - LoA_{s,[t',t]} \right) + K \right)$$

If the index is population-based (absolute) and that subject s is part of population p,

$$\Delta_a LoA_{s,t} = S \left( \beta \left( LoA_{p}^* - LoA_{s,[t',t]} \right) + K \right)$$

Where S is a scale-up parameter, $\beta$ is a parameter accounting for the population characteristics (age, sex, ethnicity, ...) determined using learning processes based on response data and/or independently measured data of the LoA, $LoA_{s,p}^*$ is the individual/subject-based reference state, $LoA_{p}^*$ is the absolute/population-based reference state using learning processes based on response data and/or independently measured data of the LoA, and K is a "translation" parameter allowing to adjust thresholds based on user preference.

In step 5 the LoAI index is generated. The value obtained in step 4 is compared with a predefined scale depending on subject/intervention characteristics determined following process described below in the section "refinement of the evaluation protocol", for example using trained machine-learning models and/or artificial intelligence, based on response data and/or independently measured data and/or reference data.

In LoAI, the index may be a population-based index, or in other words an absolute scale, in which a subject is compared with a population and/or any other suitable absolute reference. The first limit may indicate that the level of anxiety corresponds to no anxiety being experienced or being free from any anxiety as determined by the population. The second limit may indicate that the level of anxiety corresponds to experiencing the highest possible level of anxiety as determined by the population. Intermediate values represent intermediate levels of anxiety.

In LoAI, the index may be a subject-based index, or in other words a relative scale, in which each subject is compared with itself. The first limit may indicate that the level of anxiety, corresponds to no anxiety being experienced or being free from any anxiety as determined by the subject itself. The second limit may indicate that the level of anxiety corresponds to experiencing the highest possible level of anxiety as determined by the subject itself.

The method and system may determine an expected LoAI and/or ratio, for example for a point in time before, during and/or after the treatment session, for example based on the particular phase, acts of treatment already performed, etc. before, during and/or after the treatment session. For instance, if the treatment comprises a hypnotic session, an expected LoAI may be determined from the phase, for example one of phases (i) to (iv), as will be described in further detail below, of the hypnotic session, and for example from the position within such a phase. For instance, if the treatment comprises a pharmacological treatment session, an expected LoAI may be determined from the dose, titration, and/or timing of the administration of anxiolytic. The expected LoAI may for example be determined from population data or subject data, namely from historical and/or precedence data.

Because an administered treatment does not always lead to the expected LoA a difference between the measured LoA and the expected LoA provides the user with guidance for corrective measures with respect to the treatment.

It is clear that still further alternative embodiments are possible, in which there is provided a computer-implemented method for measuring, or in other words determining, and/or monitoring a level of anxiety of a subject, which comprises the method for measurement of a level of anxiety, according to one or more of the embodiments as described above, applied on a subject by receiving the measured data of a subject.

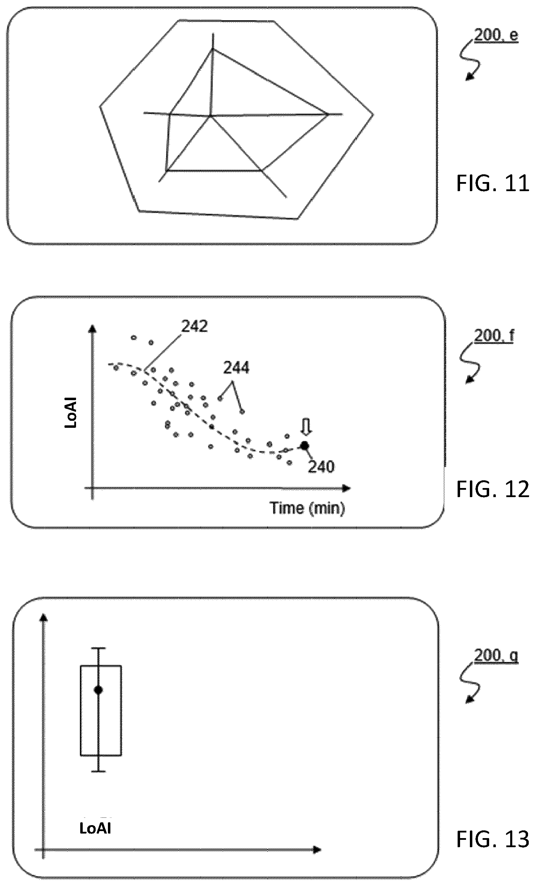

According to a further aspect, similar as described above, there is thus also provided a method for determining and/or monitoring a level of anxiety in a subject at at least two different points in time, for example before, during and/or after a treatment. According to such a method, there is measured a LoA at at least two different points in time, for example before, during and/or after the treatment. Based on a comparison of at least two LoA measured at at least two different points in time, there is then determined and/or monitored an evolution, for example a treatment-induced evolution of the LoA. However it is clear that such comparisons could be performed to determine any suitable evolution of the LoA during any suitable time period. Additionally and/or alternatively according to some embodiments the LoA could also be predicted. As for example described in further detail below with respect to Fig. 12 a suitable plurality of historical data points with respect to LoA could provide for a suitable trend analysis, for example based on a trend correlating to the a running average, mean, or any other suitable trend of the historical data points, thereby allowing prediction of future, expected or trending data points. It is clear that in this way a prediction of an evolution of the LoA can be accomplished. Predictive analysis of the historical data points, could be done by means of any suitable method, for example based on predetermined correlations between the evolution of LoA during different phases or time periods of a particular treatment, or by means of self-learning, self-optimizing algorithms, artificial intelligence algorithms, etc. which have been trained on historical data sets in order to detect repeating and/or predictable patterns and/or correlations, thereby allowing for an assessment of detectable patterns and/or correlations in the measured data, thereby allowing for a determination of current and predictive trends based on the measured data. It should be clear that alternative embodiments are possible of historical data points and/or any other suitable data resulting from LoA measurements and/or any other suitable measurement for enabling predictive analysis of current measurements. Such data on which the predictive analysis can be based can for example comprise historical data from individual subjects, from populations, predetermined treatment related patterns of an evolution of LoA, etc. According to still further embodiments, additionally and/or alternatively there may be aggregated a level of anxiety score or LoAS based on an aggregation of at least two LoA determined at at least two different points in time. Such aggregated parameters such as LoAS related to anxiety measurement could be useful in determining the impact of chronic anxiety, in which for example during a longer period, of for example several hours and/or days, the level of anxiety is continuously measured, and the aggregated LoAS is able to provide for an indicator of the impact of a constant, chronic level of anxiety as experienced by a subject as opposed to an instantaneous peak of anxiety. This is useful as even moderate or low levels of anxiety, when experienced chronically, may have a high impact on the quality of life of subjects, while for example short, infrequent bursts of anxiety, even when they are intense can be tolerated more easily. It is clear that such aggregated forms could also be useful for any parameters related to the level of anxiety as detailed in this description, and/or their scaled and/or indexed versions, such as for example LoA(I), etc. thereby allowing for similar scores of these parameters that correspond to the aggregation of at least two values of these parameters. According to particular embodiments the LoA(I)S could for example comprise any suitable embodiment of aggregation of the LoA(I) values over a predetermined time period, such as for example a sum, mean, average, etc. and the value could for example be presented in a GUI of a system similarly as described below, for example presenting the aggregated value, optionally in relation to the time period during which the LoA(I) was aggregated.

According to some treatments, such as for example anxiety treatments, in which for example a pharmacological and/or non-pharmacological treatment is used to reduce a particular level of anxiety the subject is experiencing for example by means of an anxiolytic, it is clear that the goal of the treatment is to reduce the LoA, and that such a reduction in the LoA, for example during and/or after the treatment, corresponds to a desired treatment-induced evolution of the LoA. However according to certain other treatments, for example in a context of a sedative treatment, for example prior to a chirurgical treatment, a dental treatment, etc. , it could be desired to retain the LoA as experienced in the normal awake state in which the subject does not undergo the treatment, also during the treatment. In this way, it is clear that the desired treatment induced evolution of the LoA is the prevention of an increase of the LoA, or the prevention of an increase of the LoA beyond an undesirable threshold. It is clear that according to such embodiments, the treatment-induced evolution of the LoA can for example be based on a comparison of the LoA measured before and during the treatment, or measured before and after the treatment, etc.. It is thus clear that, according to an embodiment in which the treatment is an anxiety reducing treatment, then the efficacy of such an anxiety treatment, such as for example an anxiety-reducing, anxiety-controlling, anxiety-preventing, or any other suitable anxiety treatment, can be determining and/or monitored based on the treatment-induced evolution of the LoA. In other words, when the LoA is sufficiently reduced, for example to below a desired threshold, or when the LoA does not undesirably increase, for example to above an undesired threshold. Preferably, this treatment-induced evolution of the LoA allows to optimising and/or adapting an anxiety treatment, such as for example an anxiety-reducing, anxiety-controlling, anxiety-preventing, or any other suitable anxiety treatment.

It is further also clear that based on the measured LoA, there could be determining a treatment and/or determining an adjustment to a treatment and/or determining an intensity of a treatment. For example, a higher value for the LoA could lead to a higher dosage of a sedative or anxiolytics. According to another example, if a particular anxiety treatment doesn't result in a decrease of the LoA, doesn't prevent an increase of the LoA, then an alternative treatment could be initiated. The desired level of titration of a pharmacological anxiety treatment, or the desired intensity of a non-pharmacological treatment, such as for example the depth of hypnosis, the intensity of a non-pharmacological anxiety management treatment, could for example be increased or decreased based on a desired LoA or treatment induced change thereto. According to still further embodiments, there based on a measured and/or desired LoA, there could be made a decision to choose and/or adapt and/or replace specific anxiety management treatment and/or any combination and/or mix of anxiety management treatments, such as for example one or more pharmacological and/or non-pharmacological anxiety management treatments.

The method and system may provide an output to a graphical user interface (GUI). The system may comprise a GUI. As will be described in further detail below the system may comprise a graphical user interface, GUI, configured to indicate numerically and/or graphically one or more of:

- a historic, current and/or expected LoA or LoAI;
- the evolution of and/or ratio between two LoA or LoAI measured at two different points in time;
- optionally, one or more components of the measured data, preferably one or more EEG data and optionally cardiovascular data, respiratory data, muscular activity data, ocular movement data, body temperature data, ...;
- optionally an aggregated level of anxiety score or LoAS or LoASI based on an aggregation of at least two LoA or LOAI measured at at least two different points in time.

It is clear that alternative embodiments of the GUI are possible in which further data is displayed.

The output to the GUI may additionally or alternatively show one or more current data components (e.g. one or more measured data components, one or more observed data components, and/or one or more self-reported data components). Examples of current data components include those listed elsewhere herein, preferably one or more of EEG data, cardiovascular data, respiratory data, muscular activity data, ocular movement data, body temperature data, ....

The output to the GUI may comprise a graphical indicator wherein a position and/or colour on the screen provide the user (e.g. doctor) an indication of the state of the subject and if necessary steps to be taken. For example, a green colour may indicate a positive status in terms of patient experience of signal for the user. For example, a red colour may indicate a negative status in terms of patient experience of signal for the user. For example, a left position may indicate a positive status in terms of patient experience of signal for the user. For example, a right position may indicate a negative status in terms of patient experience of signal for the user. For example, an upper position may indicate a positive status in terms of patient experience of signal for the user. For example, a lower position may indicate a negative status in terms of patient experience of signal for the user.

The output to the GUI may additionally or alternatively show one or more derived indexes. A derived index is an index derived from one or more data components and/or from another index. A derived index might be based on a ratio of LoA(I). These derived indexes may be based on collected data as observed for the subject, optionally refined using analytics (i.e. historical/population data).

A graphical output may comprise a timeline and a marker on the timeline indicating the LoAI and/or a ratio and/or an aggregation and/or a trend thereof. The LoAI and/or a ratio thereof may be on scale with a first and second limit e.g. 0 to 1, 0 to 100.

The GUI may show the current LoAI on one or more scales between the first and second limits; the scale may be linear, logarithmic, or other. The scale may be continuous, categorical, discreet, percentage, ratio.

Examples of GUIs (200, a to g) are given in FIG. 6 to FIG. 13.

FIG. 6 shows a GUI (200, a) that is a graphical display of current status of LoAI as a bar chart (252). Reference is made to the LoAI bar chart (252). The LoAI (y-axis) bar chart displayed is divided into 4 zones: 0-25% (224), 26-50% (222), 51-75% (220), 76-100% (218); these may optionally be coloured to indicate a level of safety (e.g. 76-100% - red, 51-75% - orange, 26-50% - light green, 0-25% - deep green. The 100-76% (218, red) zone may indicate that in the subject is experiencing very high, undesirable amounts of anxiety, which might for example indicate the need to optionally administer analgesic. The 75-50% (220, orange) zone may indicate that the subject is experiencing a level of anxiety that is uncomfortable. The 50-26% (222, light green) zone may indicate that the subject is experiencing a light level of anxiety. The 0-25% (224, deep green) zone may indicate that the subject is not experiencing any anxiety or a level of anxiety that is not experienced as uncomfortable. It is appreciated that the units of percent and the extent of the scale of 0 to 100% is exemplary, and other units (e.g. unitless) and scales (e.g. 0 to 1, 0 to 40, 0 to 50, 0 to 60 etc., linear, logarithmic) are within the scope of the disclosure. The height of the bar (256) is indicative of the LoAI at the indicated time (261). A numerical display (258) also indicates the LoAI. Current units (percent) are indicated (260), that can be changed (e.g. to unitless and/or other units) with a units button (262). Forwards (264, a) and backwards (264, b) buttons allow the display to scroll back and forth between current LoAI and previous readings. The percentages on the LoAI bar, might scale similarly as for an anxiety scale as mentioned above, in which for example 0% corresponds to no anxiety and 100% corresponds to the most severe anxiety possible, as experienced by the subject.

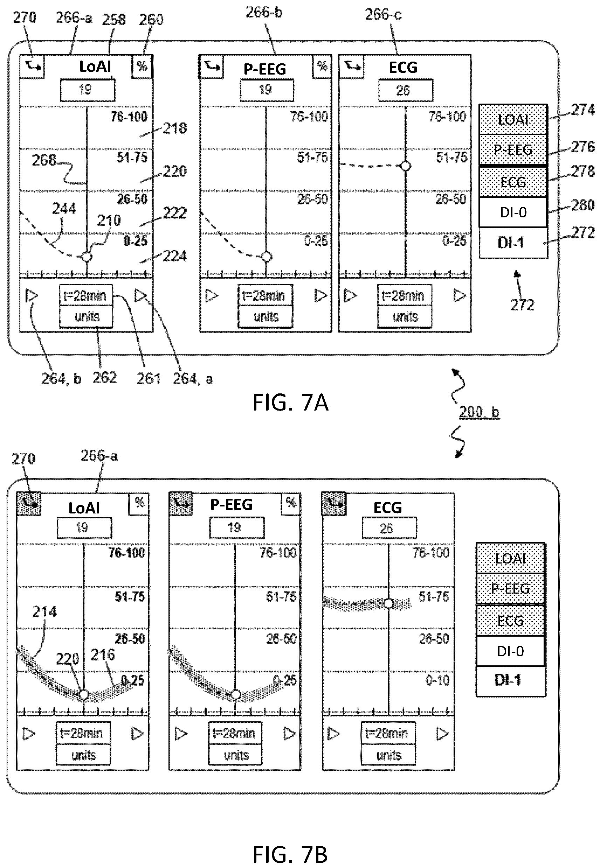

FIG.. 7A and FIG. 7B shows a GUI (200, b) that is graphical display of current status of LoAI, P-EEG and ECG as separate scroll charts (266-a 266-b, 266-c respectively). Reference is made to the LoAI scroll chart (266-a) in FIG. 7A; the other scroll charts contain equivalent features (not labelled). The LoAI scroll chart displayed is divided into 4 zones: 0-25% (224), 26-50% (222), 51-75% (220), 76-100% (218); these may optionally be coloured to indicate a level of safety (e.g. 76-100% - red, 51-75% - orange, 26-50% - light green, 0-25% - deep green as in FIG. 6. The LoAI scroll chart (266-a) contains a stationary time axis (268) along which the LoAI reading (210) at the indicated time (261) slides (up and down). A numerical display (258) also indicates the LoAI. The time axis (268) remains static, while the background scrolls in the direction of the time (typically from right to left). Historical or trending datapoints (244) are indicated. Current units (percent) are indicated (260) that can be changed (e.g. to unitless and/or other units) with a 'units' button (262). Forwards (264, a) and backwards (264, b) buttons allow the display to scroll back and forth between current LoAI and previous readings. A panel of buttons (272) allows the user to choose from a selection of scroll charts for display: buttons for LoAI (274), P-EEG (276) and ECG (280) are selected (grey background), and the selected scroll charts are displayed (266-a, 266-b, 266-c respectively). Unselected are buttons for DI-0 (278, derived index-0) and DI-1 (derived index-1, 272)).

In FIG. 7B, the same GUI as FIG. 7A are shown, and button (270) is unselected in FIG. 7A and is selected in FIG. 7B; button (270) toggles on or off a display of expected LoAI (216) for the subject that is superimposed on the scroll chart; current LoAI (210) and historical LoAI (214) can be visually compared with the expected LoAI (216).

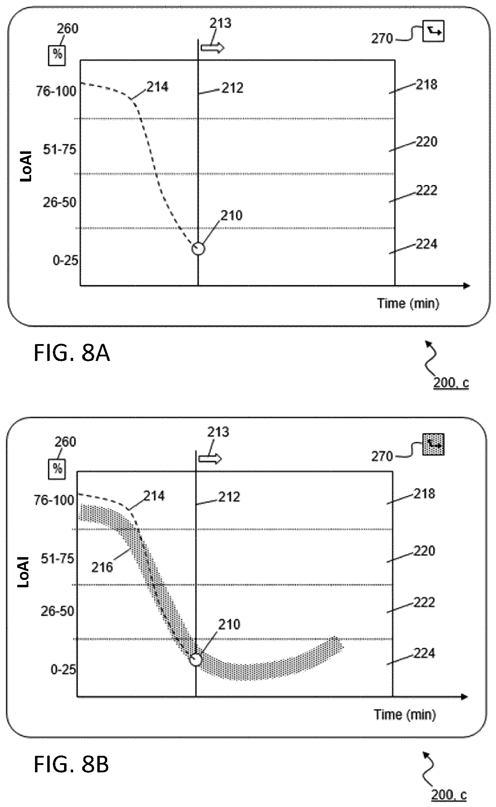

FIG. 8A and FIG. 8B shows a GUI (200, c) that is graphical display of a progress of a treatment session over time (x-axis). Reference is made to the LoAI graph in FIG. 8A; the graph in FIG. 8B contains equivalent features (not labelled). The current LoAI (210) of the subject at the current point of time shown as a circle (210) along a time axis (212) that advances in the direction of arrow (213), for example as the treatment session progresses. The LoAI graph y-axis displayed is divided into 4 zones: 0-25% (224), 26-50% (222), 51-75% (220), 76-100% (218); these may optionally be coloured to indicate a level of safety (e.g. 76-100% - red, 51-75% - orange, 26-50% - light green, 0-25% - deep green as in FIG. 7.

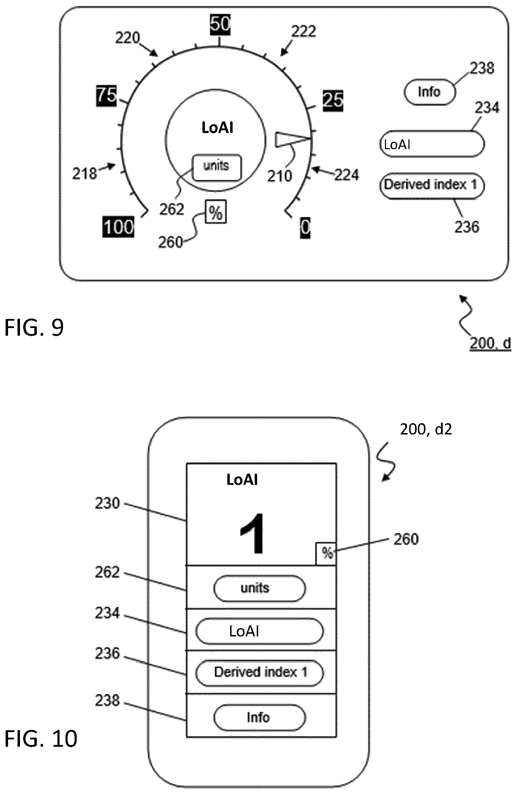

Historical or trending LoAI for the subject during the treatment session is shown as a dashed line (214). Current units (percent) are indicated (260); the box (260) may also serve as a button to cycle between different units (e.g. to unitless and/or other units). In FIG. 9B, the same graph as FIG. 9A is shown; button (270) is unselected in FIG. 9A and is selected in FIG. 9B. Button (270) toggles on or off a display of expected LoAI (216) for the subject that is superimposed on the graph; current LoAI (210) and historical LoAI (214) can be visually compared with the expected LoAI (216).

FIG. 9 shows a GUI (200, d) that contains a "speedometer" scale of the current LoAI (210) of the subject shown as a pointer. Current scale units (percent) are indicated (260), that can be changed (e.g. to unitless and/or other units) with a units button (262). The LoAI speedometer scale is divided into 4 zones: 0-25% (224), 26-50% (222), 51-75% (220), 76-100% (218); these may optionally be coloured to indicate a level of safety (e.g. 76-100% - red, 51-75% - orange, 26-50% - light green, 0-25% - deep green as in FIG. 7. Buttons (234, 236, 238) are displayed from which the user can select one or more options to change the display of the GUI and for further information.

FIG. 10 shows a GUI (200, d2) that contains a numerical display of the current LoAI (230) of the subject. Current units (percent) are indicated (260), that can be changed (e.g. to unitless and/or other units) with a units button (262). Buttons (234, 236, 238) are displayed from which the user can select one or more options to change the display of the GUI and for further information.

FIG. 11 shows a GUI (200, e) wherein different indexes are represented. External point of each axis represents the maximal scale value for this index. Chart gives an overview of the values of different indexes.

FIG. 12 shows a GUI (200, f) displaying current LoAI for the subject (240), for example during a therapeutic session, an anxiety treatment session, anxiolytic treatment, etc. over time. Historical or trending datapoints (244) are also shown, and an average of the historical or trending data points is shown as a dashed line (242).

FIG. 13 shows a GUI (200, g) displaying an average measure for the LoAI compared to the rest of the population (analytics), for example at the same point in time of the treatment session.

A system may be provided for measuring, determining and/or monitoring a Level of Anxiety of a subject, for example during a treatment session, the system comprising:

- optionally, a media renderer configured for presenting the treatment session to the subject;
- a monitoring apparatus configured to obtain measured data of the subject;

- a controller module configured for receiving measured data from the monitoring apparatus, and transforming the response data comprising the measured data into one or more of LoA(I). The evaluation protocol may comprise use of one or more of a mathematical (e.g. statistical) model, trained machine-learning model, mathematical index, reference data.

It is thus clear that, according to an embodiment, there is provided a system configured to measure a level of anxiety according to the method for measuring the LoA as described above. Such a system comprises a monitoring apparatus configured to obtain measured data comprising electroencephalogram, EEG, data collected from one or more EEG electrodes. It further comprises a controller module configured for receiving the measured data from the monitoring apparatus; extracting from the EEG data, a group 8 indicator corresponding to a power, P-power (dt), associated with a delta-theta frequency band, dt, within a delta-theta frequency range; and determining, based on said group 8 indicator, a level of anxiety, LoA, which is a value indicative for the level of anxiety in the subject. Optionally, it is clear that according to similar embodiments as explained above, the LoA could be further determined by means of group 6,7, 9, 10, 11 indicators. Preferably the monitoring apparatus comprises, for obtaining measured data one or more parietal (P) EEG electrodes configured for collection of P-EEG electrode data from the scalp anatomical region corresponding to a parietal lobe of the subject, and optionally one or more further EEG electrodes configured for collection of electroencephalogram, EEG, data, and optionally a respiratory data capturing unit, a cardiovascular data capturing unit, an muscular activity data capturing unit, a body temperature data capturing unit, etc.. It is clear, as will be explained in further detail below, that still further embodiments of the system are possible. According to such embodiments the EEG electrodes are for example also configured for collection of other data, such as for example electrocardiogram, ECG, data, any other suitable electrophysiological data related to cardiovascular data and/or respiratory data, ocular movement data, muscular activity data, ....

According to an embodiment the monitoring apparatus may be configured to capture response data of the subject, in particular measured data of the subject. The monitor apparatus comprises one or more units, each unit containing, depending on the type of unit one or more electrodes, sensors, cameras that capture measured data. The measured data component of each unit may or may not result from a processing of a signal captured by the one or more electrodes, sensors, cameras.

The monitoring apparatus may comprise an electroencephalogram (EEG) capturing unit. The response data i.e. measured data comprises outputted EEG data. EEG capturing unit may comprise at least two (e.g. 2, 3, 4, 5 or more), preferably a plurality of electrodes configured for acquiring electrical activity data from the subject brain. The electrodes may be configured for placement at predetermined positions across the subject's cephalic region; for instance to the frontal, parietal, and/or central region. An exemplary electrode configuration is 2 frontal electrodes, 2 lateral electrodes, 1 parietal electrode with respect to the head. However, it is clear that alternative embodiments are possible comprising other electrodes at other scalp locations.

In a preferred configuration, the EEG capturing unit may comprise at least three electrodes:

- the frontal EEG electrode (F-EEG electrode) configured for attachment to or contact with the scalp anatomical region corresponding to the frontal lobe;
- the parietal EEG electrode (P-EEG electrode) configured for attachment to or contact the scalp anatomical region corresponding to the parietal lobe;
- the central EEG electrode (C-EEG electrode) configured for attachment to or contact the scalp anatomical region corresponding to the precentral and postcentral gyrus.

The EEG capturing unit may further comprise a ground or reference electrode. This is situated away from the F-, P- or C-EEG electrode to allow spatial reconstruction

The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of a media renderer, as explained in further detail below. The electroencephalogram (EEG) capturing unit typically includes an amplifier for amplification of the detected signal. Signals are digitized for processing by a digital-to-analogue converter for processing by the processing unit. The sampling rate is typically at least 2.5 times higher than the highest frequency of interest. The digital-to-analogue converter may be separate or built into the processing unit.

According to an embodiment the monitoring apparatus may comprise a muscular activity capturing unit. According to an embodiment the muscular activity data capturing unit comprises an electromyography (EMG) capturing unit. The response data i.e. measured data comprises outputted muscular activity data, such as for example EMG data. An EMG capturing unit may comprise at least one (e.g. 1, 2, 3, 4, 5 or more), preferably a plurality of electrodes configured for acquiring electrical activity data from the subject muscle tissue. Where an EEG capturing unit is present, the EMG capturing unit may share at least one electrode with the EEG capturing unit. Similarly, where an EEG capturing unit is present, an EMG capturing unit may share at least one electrode with the EEG capturing unit. The electrodes may be configured for placement at predetermined positions across the subject's cranial and/or facial region; preferably to the frontal region. The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of the media renderer. The EMG capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise a respiratory data (RD) capturing unit. The response data i.e. measured data comprises outputted respiratory data. RD capturing unit may comprise at least one (e.g. 1, 2, 3, 4, 5 or more) sensor(s) configured for acquiring RD from the subject. The respiratory data may comprise one or more of respiration rate, respiration rate variability, inhalation/exhalation pressure. The RD capturing unit may comprise one or more photoplethysmogram (PPG) sensors. A PPG sensor detects blood volume changes in vasculature below the skin; the sensor is typically optical. The respiration rate and respiration rate variability may be determined from the PPG sensor. A PPG sensor may be placed at temple area and/or forehead area. The respiration rate and respiration rate variability may be determined from the PPG sensor. One or more of the sensor(s) may be configured for placement at predetermined positions across the subject's cranial and/or facial region. The sensor(s) may be integrated into a wearable device e.g. wearable headset. The sensor(s) may be fixed to or detachable from the wearable device. According to an embodiment, the sensor(s) may be dry-contact sensor(s) and/or electrode(s). The sensor(s) may be held in place be gravity, or by a force (e.g. applied by a spring or elastic) or by adhesive. The sensor(s) may be reusable or one-time use. The sensor(s) may be integrated into a head-strap or fixing band. The sensor(s) may be integrated into a mask portion of the media renderer e.g. so as to place PPG sensors at the forehead and/or temple region. The RD capturing unit may comprise a wearable chest band containing a force transducer that measures the expansion and contraction of the chest; the respiration rate and respiration rate variability may be determined from the wearable chest band sensor. The RD capturing unit may comprise an airways air pressure detector that measures the air pressure (e.g. during inhalation or exhalation). The RD capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the monitoring apparatus may comprise a cardiovascular data capturing unit. According to an embodiment the cardiovascular data capturing unit for example comprises heart rate (HR) data capturing unit. The response data i.e. measured data comprises outputted cardiovascular data. A cardiovascular data capturing unit may comprise at least one (e.g. 1, 2, 3, 4, 5 or more) sensor(s) and/or electrode(s) configured for acquiring cardiovascular data from the subject, for example in the form of an ECG. The cardiovascular data capturing unit may comprise one or more photoplethysmogram (PPG) sensors that detects blood volume changes vasculature below the skin; the sensor is typically optical. A PPG sensor may be placed at temple area and/or forehead area. The peaks of a captured PPG wave can be used to estimate cardiovascular data, such as for example the heart rate, heart rate variability as well as heartbeat interval, and blood pressure (two PPG sensors), etc.. The cardiovascular data capturing unit may comprise an ECG capturing unit comprising one or more ECG electrodes, or any other suitable electrode configured for acquiring electrical activity data from the heart of a subject. Where an EEG or EMG capturing unit is present, the ECG capturing unit may share an electrode with the EEG or EMG capturing unit. One or more of the sensor(s) and/or electrode(s) may be configured for placement at predetermined positions across the subject's cranial and/or facial region; they may be placed bilaterally. Where the sensors are PPG sensors, may be configured for placement at the forehead and/or temple region. The sensor(s) and/or electrode(s) may be integrated into a wearable device e.g. wearable headset. The sensor(s) and/or electrode(s) may be fixed to or detachable from the wearable device. The sensor(s) and/or electrode(s) may be dry-contact sensor(s) and/or electrode(s). The sensor(s) and/or electrode(s) may be held in place be gravity, or by a force (e.g. applied by a spring or elastic) or by adhesive. The sensor(s) and/or electrode(s) may be reusable or one-time use. The sensor(s) and/or electrode(s) may be integrated into a head-strap or fixing band. The sensor(s) and/or electrode(s) may be integrated into a mask portion of the media renderer e.g. so as to place PPG sensors at the forehead and/or temple region. The cardiovascular data capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise an electrodermal activity (EDA) capturing unit. The response data i.e. measured data comprises outputted EDA data. EDA capturing unit may comprise at least one (e.g. 1, 2, 3, 4, 5 or more), preferably a plurality of electrodes configured for acquiring electrical activity data from the subject skin tissue. Where an EEG or EMG capturing unit is present, the EDA capturing unit may share an electrode with the EEG or EMG capturing unit. The electrode(s) may be configured for placement at predetermined positions across the subject's cranial and/or facial region; preferably to the frontal region.

The electrode(s) may be integrated into a wearable device e.g. wearable headset. The electrode(s) may be fixed to or detachable from the wearable device. The electrode(s) may be held in place be gravity, or by a force (e.g. applied by a spring or elastic) or by adhesive. The electrode(s) may be reusable or one-time use. The electrode(s) may be integrated into a head-strap or fixing band. The electrode(s) may be integrated into a mask portion of the media renderer. The EDA capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the EDA capturing unit may be configured for measurement of galvanic skin response. The response data i.e. measured data comprises outputted GSR data. EDA capturing unit may comprise at least one (e.g. 1, 2, 3, 4, 5 or more), preferably a plurality of GSR electrodes configured for acquiring galvanic skin response. Where an EEG or EMG capturing unit is present, the EDA capturing unit may share a localized or reference electrode with the EEG or EMG capturing unit for acquiring galvanic skin response. The electrode(s) may be configured for placement at predetermined positions across the subject's cranial and/or facial region; preferably to the frontal or forehead region. The GSR electrode(s) may be integrated into a wearable device e.g. wearable headset. The GSR electrodes may be fixed to or detachable from the wearable device. The GSR electrodes may be dry-contact electrode(s). The GSR electrode(s) may be held in place be gravity, or by a force (e.g. applied by a spring or elastic) or by adhesive. The GSR electrode(s) may be reusable or one-time use. The GSR electrode(s) may be integrated into a head-strap or fixing band. The GSR electrode(s) may be integrated into a mask portion of the media renderer.

According to an embodiment, the monitoring apparatus may comprise an electrocardiogram (ECG) capturing unit. The response data i.e. measured data comprises outputted heart rate or ECG data. ECG capturing unit may comprise at least one (e.g. 1, 2, 3, 4, 5 or more), preferably a plurality of electrodes configured for acquiring electrical activity data from the subject heart. Where an EEG, EMG, or EDA capturing unit is present, the ECG capturing unit may share an electrode with the EEG, EMG, or EDA capturing unit. The electrodes being configured for placement at predetermined positions across the subject's chest, or any other suitable location where the heart ECG data can be captured. The electrodes may be integrated into a wearable device e.g. wearable headset. The electrodes may be fixed to or detachable from the wearable device. The electrodes may be held in place by gravity, or by a force (e.g. applied by a spring or elastic) or by adhesive. The electrodes may be reusable or one-time use. An electrode may be integrated into a head-strap or fixing band. An electrode may be integrated into a portion of the media renderer.

According to an embodiment, the monitoring apparatus may further comprise a physiological monitoring unit. The response data i.e. measured data comprises outputted physiological data. The physiological monitoring unit may comprise at least one (e.g. 1, 2, 3, 4 or more) sensor for acquiring subject's physiological data, in particular a component of the physiological data. The physiological data may relate to one or more of the following: pulse rate, heart rate, heart rate variability, blood pressure, respiration rate, respiration rate variability, inhalation pressure, exhalation pressure, brain oxygenation, blood O2 saturation (SpO2), regional and/or central blood O2 saturation, skin conductance, galvanic skin response, body temperature. The physiological data may relate to one or more of the following: respiration rate, respiration rate variability, inhalation pressure, heart rate.

According to an embodiment the one or more sensors may be integrated into a wearable device e.g. wearable headset. The one or more sensors may be fixed to or detachable from the wearable device. The one or more sensors may be held in place be gravity, or by a force (e.g. applied by a spring or elastic) or by adhesive. The one or more sensors may be reusable or one-time use. The one or more sensors may be integrated into a head-strap. The sensors may be integrated into a mask portion of a media renderer as explained in further detail below.

According to an embodiment, the monitoring apparatus may further comprise a body motion tracking unit. The response data i.e. measured data comprises outputted body motion tracking data. The body motion tracking unit may comprise at least one (e.g. 1, 2, 3, 4 or more) motion sensor for acquiring subject's body motion, in particular a component of the body motion tracking data. Examples of motion sensors include a 2- or 3-axis accelerometer, a gyroscope, one or more cameras, magnetic/induction transducers. The body motion includes movements of the head, limb (arms, legs, hands, knee, elbow). The body motion tracking unit may be a head movement tracking unit.

According to an embodiment the one or more motion sensors may be integrated into a wearable device e.g. wearable headset. The one or more motion sensors may be fixed to or detachable from the wearable device. The one or more motion sensors may be held in place be gravity, or by a force (e.g. applied by a spring or elastic) or by adhesive. The one or more motion sensors may be reusable or one-time use. The one or more motion sensors may be integrated into a head-strap. The motion sensors may be integrated into a mask portion of the media renderer.

According to an embodiment the monitoring apparatus may further comprise an eye-tracking unit. The response data i.e. measured data comprises outputted eye tracking data. The eye tracking unit may comprise at least one (e.g. 1, 2, 3, 4 or more) camera for monitoring movement of one or both eyes of the subject. The eye tracking unit may comprise at least one light source (e.g. visible, infrared) configured to illuminate the eye. Captures images may be analysed using eye tracking software to determine subject's focus of attention, drowsiness, consciousness or other mental states. According to an embodiment the one or more cameras may be integrated into a wearable device e.g. wearable headset. The one or more cameras may be fixed to or detachable from the wearable device. The one or more cameras may be integrated into a mask portion of the media renderer.

According to an embodiment the monitoring apparatus may further comprise a facial expression capturing unit. The response data i.e. measured data comprises outputted facial expression data - e.g. emotions, nociception. The facial expression capturing unit may comprise at least one (e.g. 1, 2, 3, 4 or more) camera for monitoring facial expressions of the subject. The facial expression capturing unit may comprise at least one light source (e.g. visible, infrared) configured to illuminate the face. Captures images may be analysed using facial expression recognition software to determine subject's facial expressions and responses, e.g. anxiety or (dis)comfort. The one or more cameras may be integrated into a wearable device e.g. wearable headset. The one or more cameras may be fixed to or detachable from the wearable device. The one or more cameras may be integrated into a portion of the media renderer.

According to particular embodiments monitoring apparatus may further comprise a body temperature data capturing unit. Body temperature data is measured data, typically measured by means of a temperature sensor, for example a temperature sensor contacting the body, however alternative embodiments are possible such as for example non-contact type sensors and/or camera's configured to measure the body temperature, for example based on infrared measurements.

According to an embodiment the monitoring apparatus may comprise an EEG capturing unit, and optionally one or more of the following: an EMG capturing unit, an EDA capturing unit, an ECG capturing unit, a physiological monitoring unit, a body temperature data capturing unit, a head tracking unit, an eye tracking unit, a face capturing unit, a respiratory data capturing unit, etc.. The monitoring apparatus according to an embodiment may comprise an EEG capturing unit, a muscular activity data capturing unit, a cardiovascular data capturing unit, an ocular movement data capturing unit, a respiratory data capturing unit, etc..

According to a particular embodiment, in which the system is used during a non-pharmacological treatment session, the system may further comprise a media renderer that presents a treatment session to the subject. The treatment session may contain hypnosis and/or other evidence-based psychological and/or mind/body intervention. The media render may comprise a screen (e.g. LED, LCD, projector) on which moving images are displayed. The images immerse the attention of the subject and/or control the subject's experience and physiological response. The media render may comprise a sound transducer (e.g. earphone, headphone, speaker) to which audio is passed (e.g. music, dialogue, sound effects).

Preferably the media renderer is integrated as a wearable device, e.g. headset. The media rendered may be provided as a virtual reality headset, as an augmented reality headset, or mixed reality headset. Most preferably the media renderer is integrated into a wearable device that provides virtual/ augmented/ mixed/ etc. reality; which typically includes a display or projector, stereo sound (mono, stereo, multidimensional), and head motion tracking sensors (e.g. gyroscopes, accelerometers, structured light systems, etc.). Examples of suitable headsets are those supplied by Oculus (e.g. Oculus Rift, Oculus Go), Pico (e.g. G2 4K, G2 Pro), LG electronics (e.g. LG 360 VR), HTC (e.g. HTC Vive), Samsung (e.g. Samsung Gear VR), Google (e.g. Google Cardboard), Microsoft (Hololens), and other off-the-shelf or customized designs. The media renderer may be expanded by also rendering somatosensory content, such as vibrations (e.g. vibration modules equipped in a seat), and/or olfactory content (e.g. releasing one or more fragrances into the nasal cavity). Optionally, the media renderer may be equipped with a computing unit for executing or playing data, or it may receive data from an external media server (i.e. streaming).

As mentioned previously, the media renderer may be integrated into a wearable device, e.g. headset. The monitoring apparatus may be integrated into the wearable device. One or more electrodes and/or one or more sensors, and/or one or more cameras of the monitoring apparatus may be integrated into the wearable device

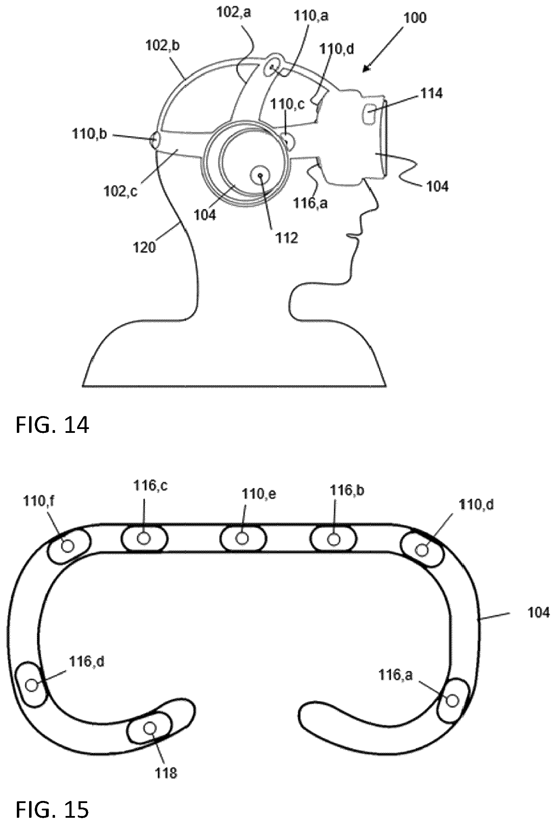

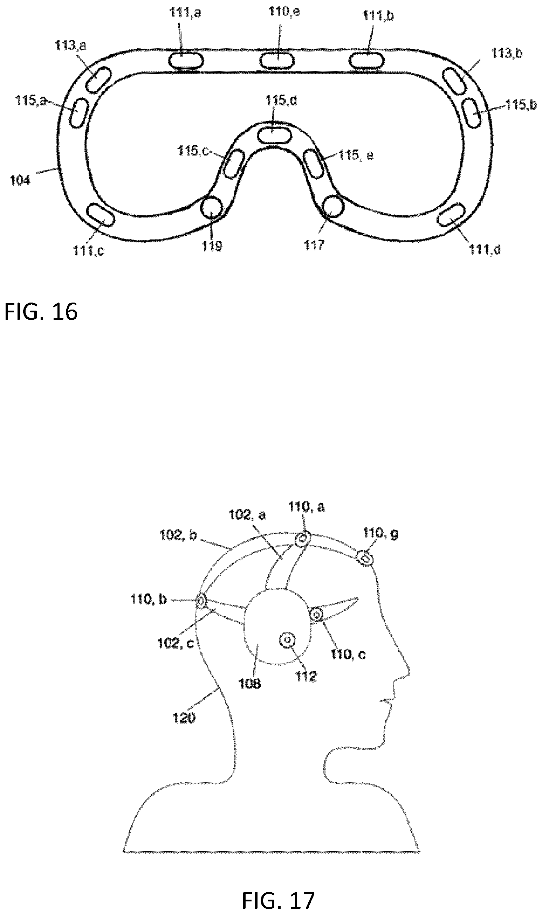

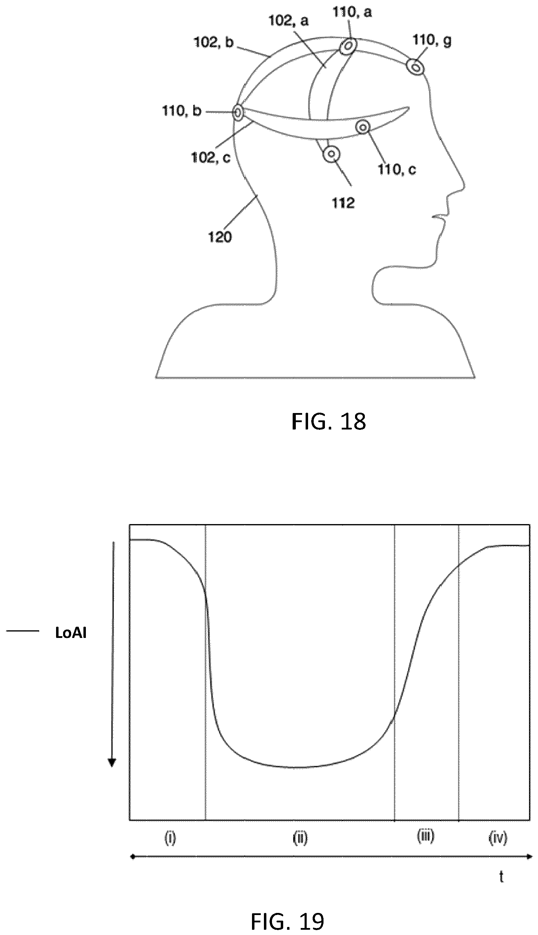

An exemplary embodiment of a wearable device is given in FIG. 14 - Fig. 18. FIG. 14 depicts an embodiment of a wearable device (100) comprising a media rendered integrated into a virtual reality viewer (104). The wearable device (100) further comprises a plurality of elasticated straps (102,a,b,c) that hold an eye mask (104) supporting a virtual reality viewer and headphones (104) in position on the head (120) of the subject. It is however clear that alternative embodiments are possible of the wearable device (100), which for example do not comprise the eye mask such as for example shown in Fig. 17 and/or the headphones such as for example shown in Fig. 18, but only the electrodes and sensors. The elasticated straps (102,a,b,c) also hold a plurality of electrodes and sensors in position on the head (120) of the subject. Depicted are electrodes (110, a (central (C) EEG electrode), b (parietal (P) EEG electrode), c (temporal), d (frontal (F) EEG electrode)) for measurement of EEG data; sensor (112) for measurement of heart rate, heart rate variability, SPO2; and camera (114) for capture of eye movement and facial expression. FIG. 15 is a view of a face-contacting edge of the embodiment of a mask disposed with electrodes (116, a, b, c, d) for measurement of EEG data; electrode (118) for measurement of skin conductance. FIG. 16 is a view of a face-contacting edge of an embodiment of the mask (104) disposed with

- a frontal (F) EEG electrode (110e) for measurement of EEG data;

- EMG electrode (111a to 111d) for measurement of EMG data;
- PPG sensors (113a, 113b) for measurement of respiratory data, cardiovascular data, blood pressure, spO2;
- EOG electrodes (115a to 115e) for measurement of EOG data;
- GSR electrode (117) for measurement of GSR data;
- ground electrode (119) that can act a reference/ground electrode in combination with one or more of the EEG (110e), EMG (111a to 111d), EOG (115a to 115e), GSR (117) electrodes.

However it is clear that the system and/or wearable device may make use of any other suitable configuration and/or arrangement of EEG electrodes, such as for example devices comprising one or more electrodes, for example two, three, four or more electrodes arranged at one or more scalp locations referred to in the international 10-20 system, or any other suitable device comprising one or more EEG electrodes. It is further also clear that the system and/or wearable device may make use of any other and/or further suitable configuration and/or arrangement of sensors and/or electrodes, such as for example a body temperature sensor, respiratory data sensor, etc.

According to an embodiment, the controller module may be configured for receiving measured data from the monitoring apparatus, and transforming the response data comprising the measured data into one or more of LoAI. The evaluation protocol may comprise use of one or more of a mathematical (e.g. statistical) model, trained machine-leaning model, mathematical index, reference data.

According to an embodiment, the controller module may be configured for receiving measured data from the monitoring apparatus, and transforming the response data comprising the measured data into the LoAI, which is representing the level of anxiety as experienced by the subject, using an evaluation protocol as described above. The evaluation protocol may for example comprise use of one or more of a mathematical (e.g. statistical) model, trained machine-leaning model, mathematical index, reference data.

According to an embodiment, the controller module typically comprises a circuit (e.g. microprocessor) configured to perform processing steps and memory. The controller module may or may not be integrated into a wearable device.

The method may be a computer implemented method.

There is further provided is a computing device or system configured for performing the method as described herein.

There is provided a computer program or computer program product having instructions which when executed by a computing device or system causing the computing device or system to perform the method as described herein.

There is provided a computer readable medium having stored thereon instructions which when executed by a computing device or system cause the computing device or system to perform the method as described herein.

There is provided a data stream which is representative of a computer program or computer program product having instructions which when executed by a computing device or system cause the computing device or system to perform the method as described herein.

The method and system described herein enable the therapist to quantify the patients level of anxiety as experienced by the subject, ... and/or enable optimal and potentially individual titration of initial dosages of analgesics, anaesthetics, anxiolytics; ... hypnotics, and/or optimize pharmacological and/or non-pharmacological sedation (VR therapy titration) and/or a mix of these; and/or provide safer progress by Quantifying, Visualizing, Trending and potentially Predicting the patient's physiological reaction / experienced level of anxiety during clinical, medical, surgical or therapeutic interventions; and/or increase subject safety.

Also provided is use of a computer-implement method for measuring LoA as described herein before, during and after a treatment providing pharmacologically and/or non-pharmacologically induced sedation of a subject. Use of the computer-implemented method for measuring LoA also functions reliably before, during and after a treatment involving sedation, which may for example be provided for a surgical intervention and/or for example replacing and/or supplementing a pharmacological sedative with a non-pharmacological sedative.

It is to be understood that this invention is not limited to particular systems and methods or combinations described, since such systems and methods and combinations may, of course, vary. It is also to be understood that the terminology used herein is not intended to be limiting, since the scope of the present invention is determined by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In this description, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The inventors have found that for the first time a strong association between response data in particular measured data collected from at least one EEG electrode, such as for example at least one of a frontal-EEG electrode, a parietal -EEG electrode and central-EEG electrode and the LoA of the subject, for example during a pharmacological and/or non- pharmacological treatment session to modify the level of anxiety experienced by the subject, such as for example by means of modifying the state of consciousness of the subject. For the first time, an objective measure of a level of anxiety of the subject can be determined in real time. This is applicable in many situations, for instance, when a subject is undergoing a surgically invasive procedure or a dental procedure under pharmacological and/or non- pharmacological sedation, specifically in case of local sedation.

State of consciousness refers to a measurement of a subject's wakefulness and/or self-awareness as well as environmental awareness determining the subject's presence sensation, arousability (emotional response) and (physical) responsiveness to an internal or external stimulus or stimuli, characterized by the physiological activity and/or by a neurological signature.

In specific state of the subject in which he experiences less or no anxiety, such as for example a specific modified state of consciousness, for example where the level of consciousness is different or modified with respect to a normal awake state, the subject for example experiences less or no anxiety and/or is optionally sedated. In such a specific state of the subject, the subject's cognitive functions may be absorbed in a specific task, and brain processes which usually happen together are separated. The creation such a specific state with reduced or no anxiety experienced by the subject, which is for example a state comprising a higher level of absorption and/or attention to a specific task, a dissociative state, or any other suitable state in which the level of consciousness is different and/or modified with respect to the normal awake state, allows a reduced perception of peripheral stimuli and a subject's modified perception of the self and the self in the environment. According to some embodiments of such a modified state of consciousness, the subject's physiology could for example also be modified (less involuntary movement i.e. swallowing, limb movement, eye blinking, higher stability of vital signs, larger respiratory patterns and improved oxygenation rates...). In such a specific state with a reduced or no experience of anxiety, such as for example a modified state of consciousness, a subject might experience partial or total catalepsy as well as disconnect from the environment (i.e. not hear/ respond to verbal command). Senses may be under/overactivated according to requirements. However, according to alternative embodiments of a specific state with reduced or no experience of anxiety, for example involving a modified state of consciousness, a subject may be absorbed into and/or directing its attention to a specific task, or in other words in a state of a higher level of absorption and/or attention. Even further embodiments are possible, in which for example the subject is subjected to for example a mind/body exercise, etc. or any other suitable physiological intervention, in which the subject is subjected to for example a purely psychological treatment, such as for example cognitive behavioural therapy, etc. For example, a subject could be induced into such a specific state with a reduced or no experience of anxiety, such as for example a modified state of consciousness, in which the modified state of consciousness, or any other suitable state, suppresses the experience of anxiety sufficiently so that it is possible to safely undergo a medical intervention, such as for example a surgical intervention, a medical intervention making use of local anaesthetics, etc..

According to an embodiment a treatment session is applied to the subject to modify the level of anxiety experienced by the subject, for example by modifying the state of consciousness of the subject. The treatment session may include one or more of hypnosis and/or other evidence-based psychological and/or mind/body intervention (i.e. non-pharmacological treatment), administration of active pharmacological ingredient (i.e. pharmacological treatment), other treatments (e.g. acupuncture, mechanical treatment, other non-pharmacological treatment). Preferably it comprises hypnosis.

In particular a non-pharmacological treatment includes mainly hypnosis but also other treatments which are used to potentialize the hypnosis session and/or decrease the level of anxiety experienced by the subject, and/or potentialize the therapeutic impact. It is clear that alternative non-pharmacological treatment sessions are possible. Examples of pharmacological treatment sessions may comprise administration of an anxiolytic, but alternative embodiments such as the administration of an analgesic and/or an anaesthetic, etc. are possible.

Examples of anxiolytics include: Barbiturates, Benzodiazepines, Carbamates, Antihistamines, Opioids, Antidepressants, Sympatholytics, etc.

Examples of analgesics and/or anaesthetics include:

An inhalation agent such as Desflurane, Enflurane, Halothane, Isoflurane, Methoxyflurane, Nitrous oxide, Sevoflurane (inhaled), etc.

An intravenous agent such as Barbiturates, Amobarbital, Methohexital, Thiamylal, Thiopental, Benzodiazepines, Diazepam, Lorazepam, Midazolam, Etomidate, Ketamine, Propofol, etc.

As used herein the term "subject" refers to the beneficiary of the therapeutic session. The "user" refers to a person or persons operating the method or system. The user may be a care provider such as a physician, or medical assistant, or non-medical assistant (e.g. friend, relative, helper) of the subject. In some circumstances, the user may be the subject, for instance, where a treatment is self-administered at home.

Hypnosis is one way of inducing an altered state of consciousness in a subject. The hypnosis therapy may be medical (clinical) hypnosis therapy, or home hypnosis therapy, or hypnosis therapy provided in any care or wellness environment. The hypnosis therapy may be extramural hypnosis therapy that is not provided in a care institution (i.e. not provided in a clinic, hospital, care centre). While immersed in the altered state of consciousness, the subject's self-perception and the peripheral awareness are affected, changing the subject's experience of his/her sensation, perception, and thoughts, and making the subject prone to follow suggestions. Subject is absorbed and dissociated from reality.

An embodiment of a hypnotic treatment session, such as for example an automated hypnotic treatment session 548 of experiment 500 as described above, typically comprises 4 sequential phases as exemplified in FIG. 19, (i) an induction phase, (ii) a deepening phase, (iii) a transition phase, and/or (iv) a re-alerting phase. While immersed in the treatment sessions the subject's self-perception and the peripheral awareness are affected, the immersion depth increasing during the (i) phase and reaching a maximum during the (ii) phase. The subject becomes prone to suggestive control during the (ii) phase, during which time the subject may be sedated, induced to relax, etc. Afterwards the subject is returned to a normal state of a consciousness by passing the (iii) and (iv) phases.

The phases are described in detail below, namely:

(i) The induction phase, wherein the subject is prepared to be immersed into the altered state. In this phase the subject is typically provided with a feeling of comfort, safety and relaxation to the subject.

(ii) The deepening phase, wherein the subject is placed in an altered state of consciousness. This state is typically characterized by full dissociation of reality and lack of or reduced movement unless expressly suggested in the therapy.

(iii) The transition phase, wherein the subject is exposed to suggestive information, which may aid in remembering or forgetting specific event of the therapy and/or to addressing one or more subject specific issues (also called post-hypnotic suggestions).

(iv) Re-alerting phase, wherein the subject is returned to a normal state of consciousness. In this phase the subject typically returns his/her senses and the dissociative state ends.

The hypnotic treatment session may be delivered by a hypnotist, more preferably it is presented using a media renderer such as a virtual reality head set for a fully immersive effect as described below in more detail. A stored hypnotic treatment session may be played through the media renderer, the content of the treatment session may be specific to the treatment goals.

While in such an altered state of consciousness, for example induced by treatments or activities inducing a higher level of absorption and/or attention, induced by hypnosis, or induced by any other suitable treatments, or alternatively any other suitable treatment not necessarily involving an altered state of consciousness, the subject can better manage anxiety as well as other symptoms/ issues. The subject can for example undergo medical interventions (e.g. invasive procedures) in the absence or with a lower dose of a pharmacological (anaesthetic / analgesic / anxiolytic) agent.

**Claims**

1. A computer-implemented method for measurement of a level of anxiety, the method comprising the steps of:

   - receiving measured data comprising EEG data collected from a parietal (P) EEG electrode configured for collection of P-EEG electrode data from the scalp anatomical region corresponding to a parietal lobe of a subject;
   - extracting from the EEG data collected from the P-EEG electrode:

     - a group 8 indicator based on a power, P-power (dt), associated with a delta-theta frequency band, dt, within a delta-theta frequency range;

   - determining, based on said group 8 indicator, a level of anxiety, LoA, which is a value indicative for the level of anxiety in the subject.

2. A method according to claim 1, wherein the delta-theta frequency band, dt, comprises one or more of the following:

   - a frequency band encompassing both delta and theta brain waves;
   - a frequency band extending from 0 Hz up to and including 8 Hz;
   - a bandwidth of at least 2 Hz and a frequency band comprising at least slow theta brain wave extending from 3 Hz up to and including 6 Hz; and/or
   wherein the delta-theta frequency range:

     - comprises a frequency range encompassing both delta and theta brain waves; and/or
     - extends from 1 Hz up to and including 6 Hz.

3. The method according to claim 1 or 2, the method comprising the steps of:

   - receiving measured data further comprising cardiovascular data
   - extracting from the cardiovascular data:

     - a group 7 indicator based on a heart rate and/or heart rate variability, preferably a combination of heart rate and heart rate variability, from the cardiovascular data;

   - determining the LoA based on said group 8 indicator and at least the group 7 indicator.

4. The method according to any of the preceding claims, the method comprising the steps of:

   - receiving measured data further comprising respiratory data;
   - extracting from the respiratory data:

     - a group 6 indicator based on respiration rate and/or a respiration variability and/or predetermined respiration patterns and/or oxygen saturation from the respiratory data;

   - determining the LoA based on said group 8 indicator and at least the group 6 indicator.

5. The method according to claim any of the preceding claims, the method comprising the steps of:

- receiving measured data further comprising

- ocular movement data;

- extracting from the ocular movement data:

- a group 9 indicator based on an ocular movement rate and/or ocular movement amplitude and/or predetermined ocular movement patterns from the ocular movement data;

- determining the LoA based on said group 8 indicator and at least the group 9 indicator, and optionally, wherein

- the ocular movement data comprises EOG data; and/or
- the group 9 indicator is based on the detection of saccadic eye movements from the ocular movement data, and optionally one or more characteristics of the saccadic eye movements.

6. The method according to claim any of the preceding claims, the method comprising the steps of:

- receiving measured data further comprising

- muscular activity data;

- extracting from the muscular activity data:

- a group 10 indicator based on a muscular activity from the muscular activity data;

- determining the LoA based on said group 8 indicator and at least the group 10 indicator, and optionally, wherein:

- the muscular activity data comprises electromyography, EMG, data; and/or
- the group 10 indicator is based on an electromyographic, EMG, activity from the EMG data.

7. The method according to claim any of the preceding claims, the method comprising the steps of:

- receiving measured data further comprising

- body temperature data;

- extracting from the body temperature data:

- a group 11 indicator based on a body temperature from the body temperature data;

- determining the LoA based on said group 8 indicator and at least the group 11 indicator.

8. The method according to any of the preceding claims, wherein the method comprises the steps of:

- receiving reference data comprising measured data of a subject and/or a population correlated to at least one predetermined reference LoA;
- determining at least one correlation of at least one predetermined reference LoA and at least one group indicator extracted from the reference data; and
- scaling and/or indexing a subsequently measured LoA with respect to the at least one predetermined reference LoA by means of said at least one correlation, and optionally determining a level of anxiety index or LoAI therefrom.

9. A computer-implemented method for determining and/or monitoring a level of anxiety of a subject, comprising the method for measurement of a level of anxiety according to any of the preceding claims applied on a subject by receiving the measured data of a subject.

10. A computer-implemented method for determining and/or monitoring and/or predicting and/or aggregating a level of anxiety in a subject before, during and/or after a treatment, wherein the method comprises the steps of:

- measurement of the LoA according to any of the preceding claims, at at least two different points in time, preferably before, during and/or after the treatment;
- determining and/or monitoring and/or predicting and/or aggregating an evolution, preferably a treatment-induced evolution of the LoA, based on a comparison of at least two LoA measured at at least two different points in time; and/or
- aggregating a level of anxiety score or LoAS based on an aggregation of at least two LoA measured at at least two different points in time.

11. The method according to claim 10, wherein the method comprises the step of:

- determining the treatment-induced evolution of the LoA, based on a comparison of the LoA measured during and/or after the treatment with the LoA measured before the treatment.

12. The method according to claim 10 or 11, wherein the treatment is an anxiety reducing treatment and the method comprises the step of:

- determining and/or monitoring the efficacy of the anxiety reducing treatment based on the treatment-induced evolution of the LoA; and/or
- optimizing and/or adapting an anxiety reducing treatment based on the treatment-induced evolution of the LoA.

13. A computer-implemented method for determining a treatment and/or determining an adjustment to a treatment and/or determining an intensity of a treatment, wherein the method comprises the steps of:

- measuring a LoA according to any of the claims 1 to 8;
- determining a treatment and/or determining an adjustment to a treatment and/or determining an intensity of a treatment based on the measured LoA.

14. A system configured to measure a level of anxiety according to the method according to any of the preceding claims, the system comprising:

- a monitoring apparatus configured to obtain measured data comprising electroencephalogram, EEG, data comprising P-EEG data collected from a P-EEG electrode;
- a controller module configured for:
- receiving the measured data from the monitoring apparatus;
- extracting from the EEG data:

- the group 8 indicator based on a power, P-power (dt), associated with a delta-theta frequency band, dt, within a delta-theta frequency range;

- determining, based on said group 8 indicator, the level of anxiety, LoA, which is a value indicative for the level of anxiety in the subject.

15. A system according to claim 14, wherein the monitoring apparatus comprises, for obtaining measured data, one or more of:

- one or more parietal (P) EEG electrodes configured for collection of P-EEG electrode data from the scalp anatomical region corresponding to a parietal lobe of the subject,
- optionally, one or more further EEG electrodes configured for collection of electroencephalogram, EEG, data;
- optionally, one or more frontal (F) EEG electrodes configured for collection of F-EEG electrode data from the scalp anatomical region corresponding to a frontal lobe of the subject,
- optionally, one or more central (C) EEG electrodes configured for collection of C-EEG electrode data from the scalp anatomical region corresponding to a precentral and postcentral gyrus of the subject;
- optionally a respiratory data capturing unit;
- optionally a cardiovascular data capturing unit;
- optionally an ocular movement data capturing unit;
- optionally a muscular activity data capturing unit;
- optionally a body temperature data capturing unit; and/or

wherein the system further comprises a graphical user interface, GUI, configured to indicate numerically and/or graphically one or more of:

- at least two LoA and/or LoAI respectively measured at different points in time, preferably a historic, current and/or expected LoA and/or LoAI;
- the evolution of and/or ratio between and/or aggregation of at least two LoA and/or LAI measured at different points in time;
- optionally an aggregated level of anxiety score or LoAS or LoAIS based on an aggregation of at least two LoA or LoAI measured at at least two different points in time;
- optionally, one or more components of the measured data, preferably one or more P-EEG electrode data.

FIG. 1

FIG. 2

```
                                                            ┌─── 300
     ┌ ─ ─ ─ ─ ─ ─ ─ ─ ─ ─ ─ ─ ─ ─ ─ ─ ┐
     │   ┌─────────────────────────────┐  │
     │   │      Receive P-EEG data     │ ─┼── 302
     │   └─────────────────────────────┘  │
     │                 │                  │
     │                 ▼                  │
     │   ┌─────────────────────────────┐  │
     │   │   Extract group 8 indicator: │ ─┼── 304
     │   │        P-Power(dt)          │  │
     │   └─────────────────────────────┘  │
     │                 │                  │
     │                 ▼                  │
     │   ┌─────────────────────────────┐  │
     │   │       Determine LoA         │ ─┼── 306
     │   └─────────────────────────────┘  │
     └ ─ ─ ─ ─ ─ ─ ─ ─ ─ ─ ─ ─ ─ ─ ─ ─ ┘
```

## FIG. 3

```
        ╔══════════════════════════════════════╗
        ║            treatment          ──────╲ ║      ┌── 400
        ╚═════════╤════════════════════════════╝╱
     ┌ ─ ─ ─ ─ ─ ─│─ ─ ─ ─ ─ ─ ─ ─ ─│─ ─ ─ ─ ─ ─ ─ ─ ─ ─ ─ ┐
        408 ──────┘           ┌── 402 │          ┌ ─ ─ ─ ─ ─ ┐
     │                        │       ▼                      │
        ┌────────────────────┐│   ┌────────────────────┐  ┆── 410
     │  │   Measure LoA      │──► │   Measure LoA       │  ┆       │
        │ at first point in  │    │ At second point in  │  └ ─ ─ ─ ─
     │  │      time          │    │      time           │ ─── 404  │
        └────────────────────┘    └────────────────────┘
     │        ▲  │                       │   ▲  └─── 300       │
          300 ─┘  │                      │   │
     │            │      ┌───────────────────────┐               │
                  └──────│   Determine treatment- │ ── 406
     │                   │  induced evolution of LoA │            │
                         └───────────────────────┘
     └ ─ ─ ─ ─ ─ ─ ─ ─ ─ ─ ─ ─ ─ ─ ─ ─ ─ ─ ─ ─ ─ ─ ─ ─ ─ ┘
```

## FIG. 4

FIG. 5

FIG. 6

FIG. 7A

FIG. 7B

FIG. 8A

FIG. 8B

FIG. 9

200, d

FIG. 10

200, d2

FIG. 11

200, e

FIG. 12

200, f

FIG. 13

200, g

FIG. 14

FIG. 15

FIG. 16

FIG. 17

FIG. 18

FIG. 19

FIG. 20

FIG. 21

FIG. 22

FIG. 23

FIG. 24

FIG. 25

FIG. 26

FIG. 27

FIG. 28

FIG. 29

FIG. 30

EP 3 954 290 A1

FIG. 31

FIG. 32

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 107 368 692 A (SHENZHEN INST ADV TECH) 21 November 2017 (2017-11-21) * paragraphs [0069], [0072], [0075] - [0077], [0080] - [0082]; figures 2A, 4 * ----- | 1-15 | INV. A61B5/16 A61B5/374 G16H20/10 G16H50/20 A61M21/00 |
| X | US 2014/200432 A1 (BANERJI SUBHASIS [SG] ET AL) 17 July 2014 (2014-07-17) * paragraphs [0013], [0114], [0115], [0119], [0161], [0171], [0188], [0200]; figures 1,2 * ----- | 1-15 | ADD. A61B5/01 A61B5/024 A61B5/0245 |
| A | US 2008/177197 A1 (LEE KOOHYOUNG [US] ET AL) 24 July 2008 (2008-07-24) * paragraphs [0022] - [0048]; figures 1-14 * ----- | 1-15 | A61B5/08 A61B5/145 |

TECHNICAL FIELDS
SEARCHED      (IPC)

A61B

The present search report has been drawn up for all claims

| Place of search | Date of completion of the search | Examiner |
|---|---|---|
| The Hague | 25 January 2021 | Mecking, Nikolai |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

 
& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 20 19 0368

This annex lists the patent family members relating to the patent documents cited in the above-mentioned European search report.
The members are as contained in the European Patent Office EDP file on
The European Patent Office is in no way liable for these particulars which are merely given for the purpose of information.

25-01-2021

| Patent document cited in search report | | Publication date | Patent family member(s) | | Publication date |
|---|---|---|---|---|---|
| CN 107368692 | A | 21-11-2017 | NONE | | |
| US 2014200432 | A1 | 17-07-2014 | AU | 2012259507 B2 | 25-08-2016 |
| | | | CN | 103764021 A | 30-04-2014 |
| | | | EP | 2709522 A1 | 26-03-2014 |
| | | | MY | 167168 A | 13-08-2018 |
| | | | PL | 2709522 T3 | 31-03-2017 |
| | | | PT | 2709522 T | 30-11-2016 |
| | | | US | 2014200432 A1 | 17-07-2014 |
| | | | US | 2020237618 A1 | 30-07-2020 |
| | | | WO | 2012161657 A1 | 29-11-2012 |
| US 2008177197 | A1 | 24-07-2008 | AU | 2007345266 A1 | 31-07-2008 |
| | | | CA | 2675507 A1 | 31-07-2008 |
| | | | CN | 101677774 A | 24-03-2010 |
| | | | EP | 2120692 A1 | 25-11-2009 |
| | | | IL | 199953 A | 28-11-2013 |
| | | | JP | 5373631 B2 | 18-12-2013 |
| | | | JP | 2010516329 A | 20-05-2010 |
| | | | KR | 20100014815 A | 11-02-2010 |
| | | | US | 2008177197 A1 | 24-07-2008 |
| | | | WO | 2008091323 A1 | 31-07-2008 |

EPO FORM P0459

For more details about this annex : see Official Journal of the European Patent Office, No. 12/82